# EUROPEAN PATENT APPLICATION

(11) **EP 2 666 356 A1**
(43) Date of publication of application: **27.11.2013**
(21) Application number: 13176166.0
(22) Date of filing: 12.06.2009
(51) Int. Cl.: A01N 33/02, A61K 31/135, A61P 25/16

(54) **Rasagiline for parkinson's disease modification**

(30) Priority: 13.06.2008 US 131936 P; 22.08.2008 US 189724 P
(62) Divisional of application: 09762921.6
(71) Applicant: TEVA PHARMACEUTICAL INDUSTRIES, LTD., 49131 Petah Tiqva (IL)
(72) Inventor: Levy, Ruth, 69620 Tel Aviv (IL); Eyal, Eli, 49321 Petach-Tikva (IL); Goren, Tamar, 76310 Rehovot (IL); Oren, Sheila, 46301 Herzliya (IL); Sayag, Naim, 20142 Yuvalim (IL); Weiss, Yonatan, 30900 Zichron Yaacov (IL); Ben-Ami, Miri, 49726 Petach-Tikva (IL)
(74) Representative: Nachshen, Neil Jacob

(57) **Abstract**

A method for modifying Parkinson's disease by periodically administering a pharmaceutical composition comprising a therapeutically effective amount of rasagiline or a pharmaceutically acceptable salt of rasagiline to the patient, thereby modifying the disease. The method includes reducing the rate of progression; delaying the need for symptomatic anti-Parkinsonian therapy; reducing the risk of a Parkinson's disease patient requiring symptomatic anti-Parkinsonian therapy; and reducing the functional decline.

## Description

This application claims benefit of U.S. Provisional Application Nos. 61/189,724, filed August 22, 2008 and 61/131,936, filed June 13, 2008, the contents of which are hereby incorporated by reference.

Throughout this application various publications are referenced. The disclosures of these publications in their entireties are hereby incorporated by reference into this application in order to more fully describe the state of the art to which this invention pertains.

### Background of the Invention

Parkinson's disease (PD) is the second most common age-related neurodegenerative disorder, affecting 1-2% of persons over the age of 60 years. Current therapies are primarily based on a dopamine replacement strategy (Olanow CW, Watts RL, Koller WC. An algorithm (decision tree) for the management of Parkinson's disease (2001): treatment guidelines. Neurology 2001; 56(suppl 5): 1-88; Rascol O, Goetz C, Koller W, Poewe W, Sampaio C. Treatment interventions for Parkinson's disease: an evidence based assessment. Lancet. 2002 359:1589-1598). However, chronic levodopa treatment is associated with the development of potentially disabling motor complications in up to 90% of patients (Ahlskog JE, Muenter MD. Frequency of levodopa-related dyskinesias and motor fluctuations as estimated from the cumulative literature. Mov Disord. 2001, 16:448-58). In addition, potentially disabling features such as gait dysfunction, loss of balance, freezing, sleep disturbances, autonomic disorders, and dementia are often not satisfactorily controlled with available therapies (Lang AE, Obeso JA. Time to move beyond nigrostriatal dopamine deficiency in Parkinson's disease. Ann Neurol 2004, 55: 761-765). These levodopa non-responsive features are thought to reflect non-dopaminergic pathology in the brain, spinal cord, and peripheral autonomic nervous system (Forno LS. Neuropathology of Parkinson's disease. J Neuropathol Exp Neurol. 996;55:259-272; Braak H, Tredici KD, Rub U, de Vos RA, Jansen Steur EN, Braak E. Staging of brain pathology related to sporadic Parkinson's disease. Neurobiol Aging 2003, 24:197-211). Indeed, prospective long-term studies indicate that levodopa non-responsive features are the primary source of disability and nursing home placement in advanced patients (Hely MA, Morris JG, Traficante R, Reid WG, O'Sullivan DJ, Williamson PM. The Sydney multicentre study of Parkinson's disease: progression and mortality at 10 years. J Neurol Neurosurg Psychiatry. 1999, 67:300-307). Thus, many PD patients suffer disability despite currently available therapies. The development of a neuroprotective therapy that slows, stops, or reverses disease progression is the highest priority in PD research.

Clinical trials have examined several promising compounds to determine if they have disease modifying effects in PD (Schapira AH, Olanow CW. Neuroprotection in Parkinson disease: mysteries, myths, and misconceptions. JAMA. 2004, 291:358-364). Several were negative showing no effect of the study drug on the outcome measure; however some were positive but could not be established to be neuroprotective because of the possibility that the outcome measure was confounded by a symptomatic or pharmacologic effect of the study intervention. In a study, the primary endpoint was the time to development of disability necessitating levodopa therapy (Parkinson's Study Group. Effects of tocopherol and deprenyl on the progression of disability in early Parkinson's disease. N Engl JMed 1993, 328:176-183). However, this study could not determine if positive results in this trial were due to selegiline having a protective effect that slowed degeneration or a symptomatic effect that masked it (Olanow CW, Calne D. Does selegiline mono therapy in Parkinson's Disease act by symptomatic or protective mechanisms? Neurology 1991, 42:41-48). In another study, the primary endpoint was the change in Unified Parkinson's Disease Rating Scale (UPDRS) motor score between untreated baseline and an untreated final visit performed after 12 months of treatment with selegiline or placebo and 2 months of drug wash-out (Olanow CW, Hauser RA, Gauger L, et al. The effect of deprenyl and levodopa on the progression of signs and symptoms in Parkinson's disease. Ann Neurol 1995, 38:771-777). In this study, patients treated with selegiline had less deterioration from baseline than patients treated with placebo, but a confounding long-duration symptomatic effect lasting longer than 2 months could not be excluded.

More recently, a novel propargylamine ("TCH346") was studied as a possible neuroprotective agent. In the laboratory, TCH346 was observed to have protective effects in both *in vitro and in vivo* models, even when administered in low doses (Waldmeier PC, Boulton AA, Cools AR, Kato AC, Tatton WG. Neurorescuing effects of the GAPDH ligand CGP 3466B. J Neural Transm Suppl. 2000, (60):197-214; Andringa G, van Oosten RV, Unger W et al, Systemic administration of the propargylamine CGP 3466B (TCH346) prevents behavioural and morphological deficits in rats with 6-hydroxydopamine-induced lesions in the substantia nigra. Eur J Neurosci 2000, 12:3033-3043; Andringa G, Eshuis S, Perentes E. TCH346 prevents motor symptoms and loss of striatal FDOPA uptake in bilaterally MPTP-treated primates. Neurobiol Dis 2003; 14: 205-217). As TCH346 did not inhibit Type-B Monoamine Oxidase (MAO-B), there was optimism that the drug would not be confounded by symptomatic effects. The drug was tested in a prospective, double-blind, placebo-controlled multi-center trial using time to need for levodopa as the primary endpoint (Olanow CW, Schapira AH, LeWitt PA, Kieburtz K, Sauer D, Olivieri G, Pohlmann H, Hubble J. TCH346 as a neuroprotective drug in Parkinson's disease: a double-blind, randomised, controlled trial. Lancet Neurol. 2006, 5:1013-1020). At the three doses tested, TCH346 did not demonstrate a positive effect on any of the primary or secondary endpoints, and did not demonstrate a disease modifying effect.

### Summary of the Invention

The subject invention provides a method of reducing the rate of progression of Parkinson's disease symptoms in an early stage Parkinson's disease patient, the method comprising identifying an early stage Parkinson's disease patient, and periodically administering to the early stage Parkinson's disease patient so identified an amount of rasagiline, or a pharmaceutically acceptable salt of rasagiline effective to reduce the rate of progression of Parkinson's disease symptoms.

The subject invention also provides a method of reducing the rate of progression of Parkinson's disease symptoms in a Parkinson's disease patient, the method comprising periodically administering to the Parkinson's disease patient for more than 52 weeks an amount of rasagiline, or a pharmaceutically acceptable salt of rasagiline effective to reduce the rate of progression of Parkinson's disease symptoms.

The subject invention further provides a method for delaying the need for symptomatic anti-Parkinsonian therapy in an early stage Parkinson's disease patient, comprising identifying a patient to be an early stage Parkinson's disease patient, and periodically administering to the patient so identified an amount of rasagiline or a pharmaceutically acceptable salt of rasagiline effective to delay the need for symptomatic anti-Parkinsonian therapy.

The subject invention yet further provides a method for reducing the risk of a Parkinson's disease patient requiring symptomatic anti-Parkinsonian therapy, comprising periodically administering to the patient for 36 weeks an amount of rasagiline or a pharmaceutically acceptable salt of rasagiline effective to reduce the risk of requiring symptomatic anti-Parkinsonian therapy.

The subject invention yet further provides a method of reducing the functional decline of an early stage Parkinson's disease patient, comprising identifying a patient to be an early stage Parkinson's disease patient, and periodically administering to the patient so identified an amount of rasagiline or a pharmaceutically acceptable salt of rasagiline effective to reduce the functional decline.

The subject invention yet further provides a method of reducing the functional decline in a Parkinson's disease patient, comprising periodically administering to the patient for more than 52 weeks an amount of rasagiline or a pharmaceutically acceptable salt of rasagiline effective to reduce the functional decline.

The subject invention yet further provides a method of treating a patient exhibiting early signs of Parkinson's disease, comprising identifying a patient exhibiting early signs of Parkinson's disease, and periodically administering to the patient so identified an amount of rasagiline or a pharmaceutically acceptable salt of rasagiline effective to treat the patient.

The subject invention yet further provides a method of reducing the fatigue in an early stage Parkinson's disease patient, comprising identifying a patient to be an early stage Parkinson's disease patient, and periodically administering to the patient so identified an amount of rasagiline or a pharmaceutically acceptable salt of rasagiline effective to reduce fatigue.

The subject invention yet further provides a method of reducing the severity of non-motor symptoms in an early stage Parkinson's disease patient, comprising identifying a patient to be an early stage Parkinson's disease patient, and periodically administering to the patient so identified an amount of rasagiline or a pharmaceutically acceptable salt of rasagiline effective to reduce the severity of non-motor symptoms.

The subject invention further provides a method of reducing fatigue in an early stage Parkinson's disease patient, comprising periodically administering to an early stage Parkinson's disease patient an amount of rasagiline or a pharmaceutically acceptable salt of rasagiline effective to reduce fatigue.

The subject invention further provides a method of reducing severity of non-motor symptoms in an early stage Parkinson's disease patient, comprising periodically administering to an early stage Parkinson's disease patient an amount of rasagiline or a pharmaceutically acceptable salt of rasagiline effective to reduce the severity of non-motor symptoms.

The subject invention further provides a method of slowing clinical progression and treating symptoms of Parkinson's disease in a Parkinson's disease patient comprising periodically administering to the Parkinson's disease patient an amount of rasagiline or a pharmaceutically acceptable salt of rasagiline, effective to slow clinical progression and treat the signs and symptoms of Parkinson's disease in the patient.

The subject invention further provides rasagiline or a pharmaceutically acceptable salt of rasagiline for use in reducing the rate of progression of Parkinson's disease symptoms in an early stage Parkinson's disease patient.

The subject invention further provides rasagiline or a pharmaceutically acceptable salt of rasagiline for use in delaying the need for symptomatic anti-Parkinsonian therapy in an early stage Parkinson's disease patient.

The subject invention further provides rasagiline or a pharmaceutically acceptable salt of rasagiline for use in reducing the risk of an early stage Parkinson's disease patient requiring symptomatic anti-Parkinsonian therapy.

The subject invention further provides rasagiline or a pharmaceutically acceptable salt of rasagiline for use in reducing the functional decline in an early stage Parkinson's disease patient.

The subject invention further provides rasagiline or a pharmaceutically acceptable salt of rasagiline for use in treating a patient exhibiting early signs of Parkinson's disease.

The subject invention further provides rasagiline or a pharmaceutically acceptable salt of rasagiline for use in reducing the fatigue in an early stage Parkinson's disease patient.

The subject invention further provides rasagiline or a pharmaceutically acceptable salt of rasagiline for use in reducing the severity of non-motor symptoms in an early stage Parkinson's disease patient.

The subject invention yet further provides a pharmaceutical composition comprising a pharmaceutically effective amount of rasagiline or a pharmaceutically acceptable salt of rasagiline for use in reducing the rate of progression of Parkinson's disease symptoms in an early stage Parkinson's disease patient.

The subject invention yet further provides a pharmaceutical composition comprising a pharmaceutically effective amount of rasagiline or a pharmaceutically acceptable salt of rasagiline for use in delaying the need for symptomatic anti-Parkinsonian therapy in an early stage Parkinson's disease patient.

The subject invention yet further provides a pharmaceutical composition comprising a pharmaceutically effective amount of rasagiline or a pharmaceutically acceptable salt of rasagiline for use in reducing the risk of an early stage Parkinson's disease patient requiring symptomatic anti-Parkinsonian therapy.

The subject invention yet further provides a pharmaceutical composition comprising a pharmaceutically effective amount of rasagiline or a pharmaceutically acceptable salt of rasagiline for use in reducing the functional decline in an early stage Parkinson's disease patient.

The subject invention yet further provides a pharmaceutical composition comprising a pharmaceutically effective amount of rasagiline or a pharmaceutically acceptable salt of rasagiline for use in treating a patient exhibiting early signs of Parkinson's disease.

The subject invention yet further provides a pharmaceutical composition comprising a pharmaceutically effective amount of rasagiline or a pharmaceutically acceptable salt of rasagiline for use in reducing the fatigue in an early stage Parkinson's disease patient.

The subject invention yet further provides a pharmaceutical composition comprising a pharmaceutically effective amount of rasagiline or a pharmaceutically acceptable salt of rasagiline for use in reducing the severity of non-motor symptoms in an early stage Parkinson's disease patient.

### Brief Description of Figures

The patent or application file contains at least one drawing executed in color. Copies of this patent or patent application publication with color drawing(s) will be provided by the Office upon request and payment of the necessary fee.
- Figure 1.: ADAGIO Study Design

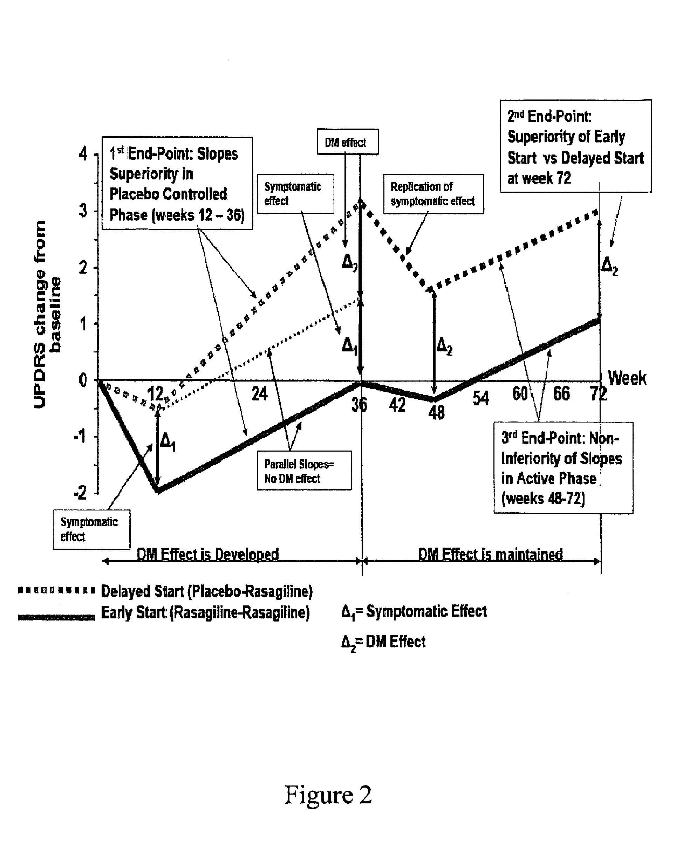

- Figure 2.: ADAGIO: Disease Modification Principal Statistical Analyses - 3 Primary Analyses

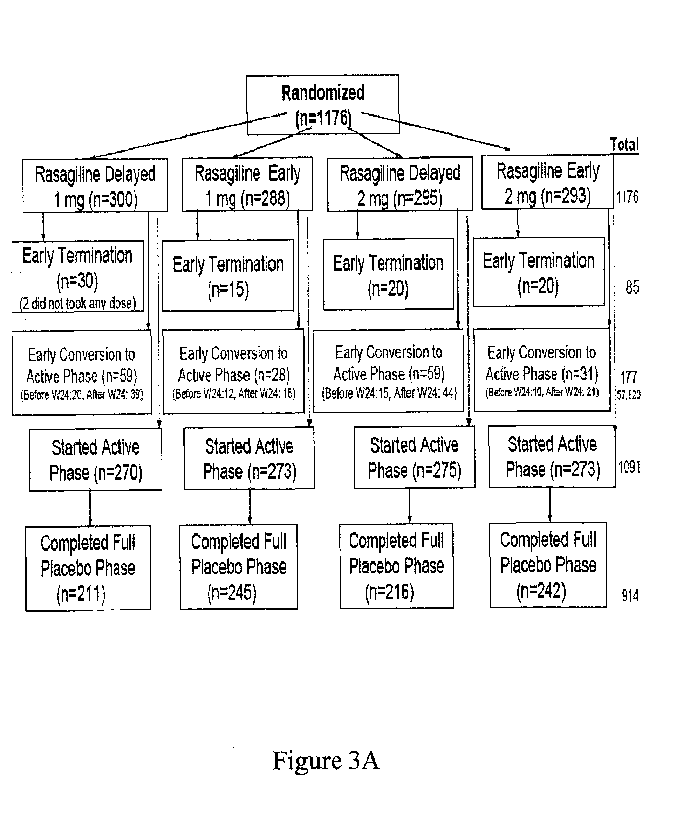

- Figure 3A.: Subjects Disposition: Placebo-Controlled ("PC") Phase

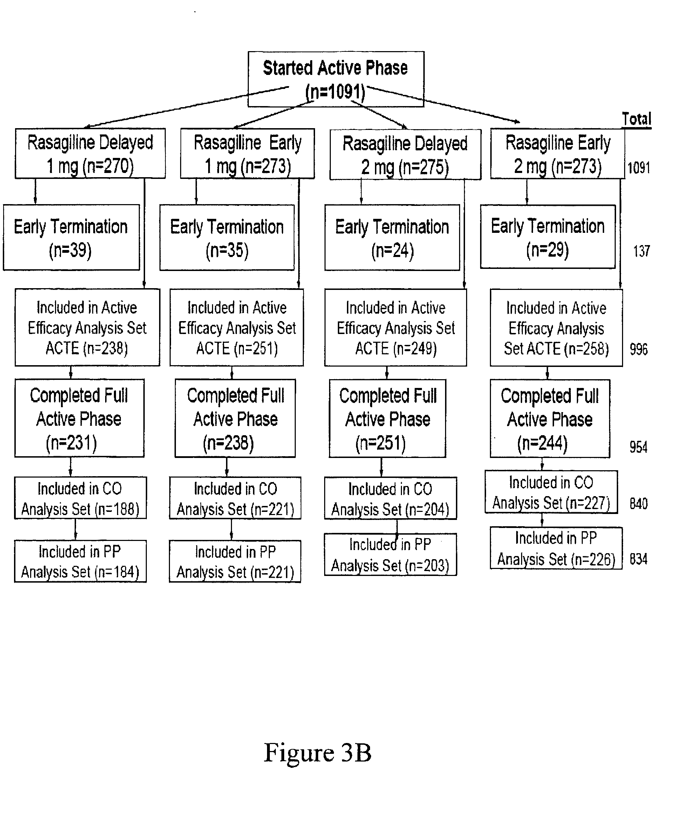

- Figure 3B.: Subjects Disposition: Active Treatment Phase

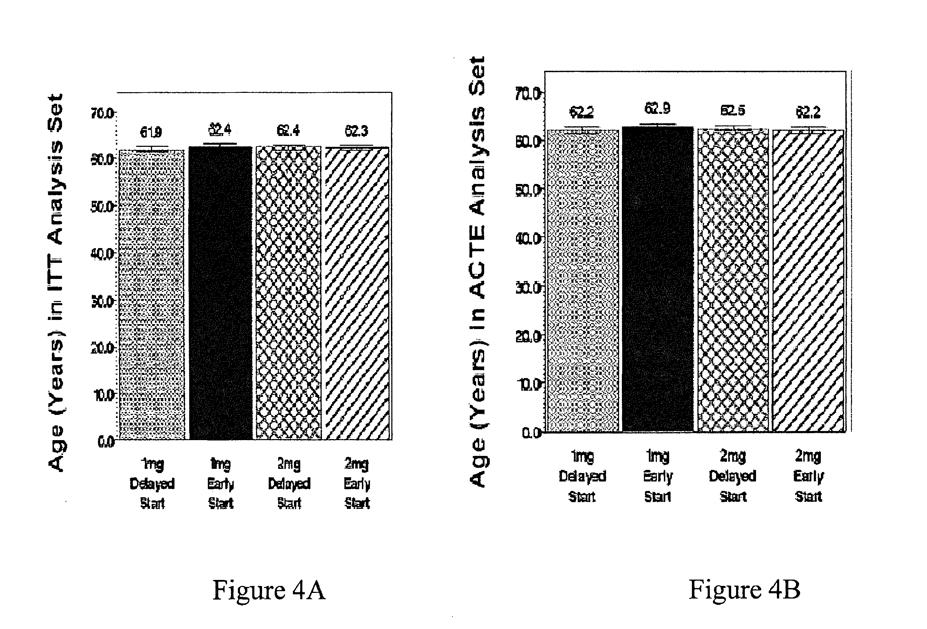

- Figure 4A.: Mean (SE) of Age (Years): ITT Analysis Set (N=1174)
- Figure 4B.: Mean (SE) of Age (Years): Active Efficacy (ACTE) Analysis Set (N=996)

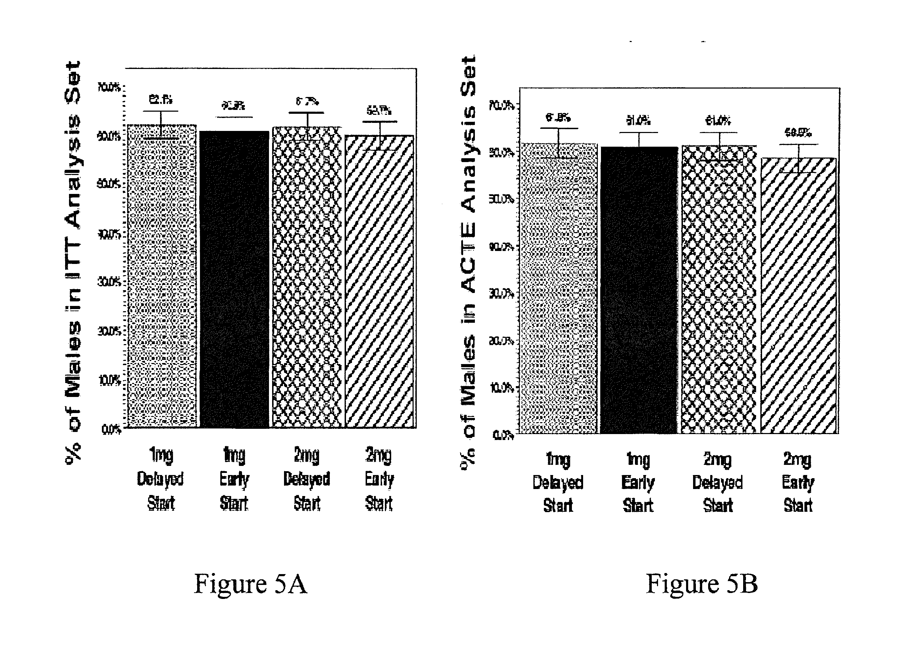

- Figure 5A.: % (SE) of Males: ITT Analysis Set (N=1174)
- Figure 5B.: % (SE) of Males: Active Efficacy (ACTE) Analysis Set (N=996)

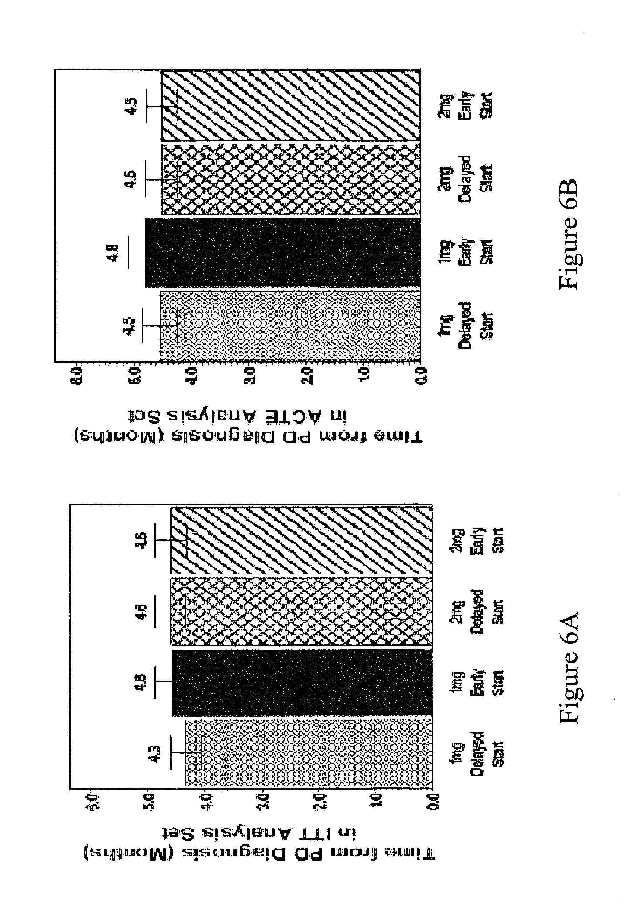

- Figure 6A.: Mean (SE) of Time from PD Diagnosis (Months): ITT Analysis Set (N=1174)
- Figure 6B.: Mean (SE) of Time from PD Diagnosis (Months): Active Efficacy (ACTE) Analysis Set (N=996)

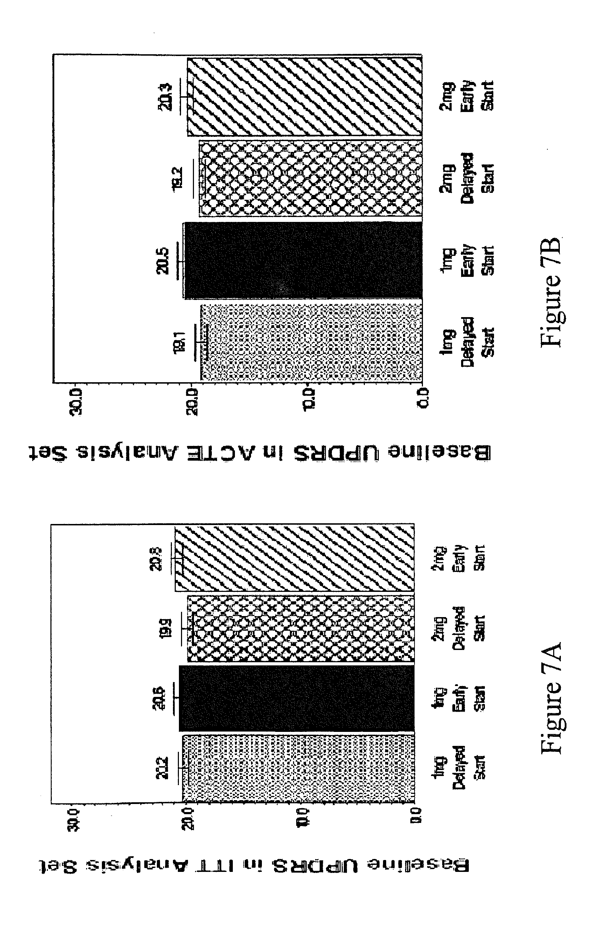

- Figure 7A.: Mean (SE) of Total UPDRS Score at Baseline: ITT Analysis Set (N=1174)
- Figure 7B.: Mean (SE) of Total UPDRS Score at Baseline: Active Efficacy (ACTE) Analysis Set (N=996)

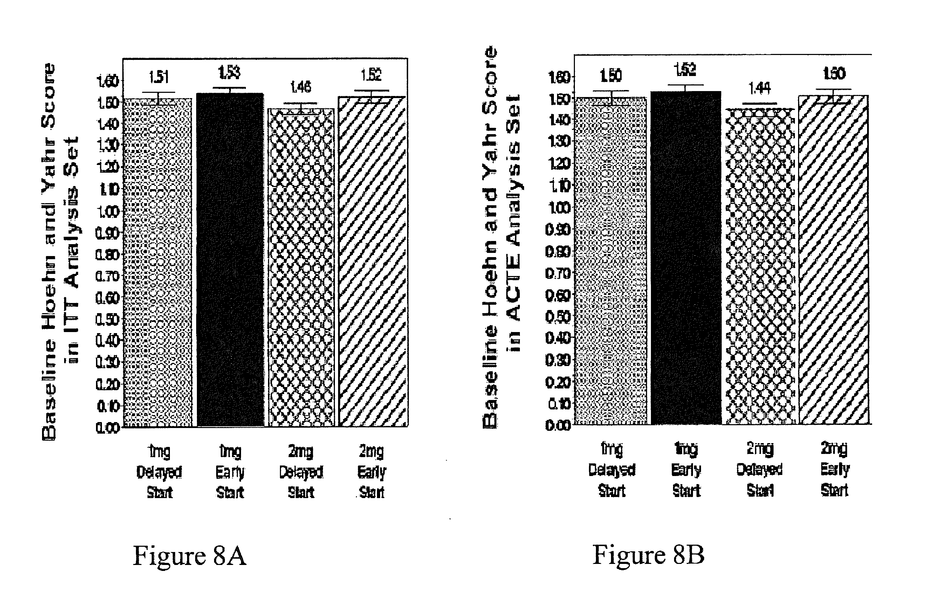

- Figure 8A.: Mean (SE) of Hoehn and Yahr Score at Baseline: ITT Analysis Set (N=1174)
- Figure 8B.: Mean (SE) of Hoehn and Yahr Score at Baseline: Active Efficacy (ACTE) Analysis Set (N=996)
- Figure 9.: Results of Primary analysis #1 - Comparison of Slopes in the PC phase (weeks 12 - 36): TVP-1012/500 (ADAGIO) Mean + - SE of Change in Total UPDRS Score - ITT Data Analysis Set at PC Phase
- Figure 10A.: Results of Primary analysis #1 - Comparison of Slopes in the PC phase (weeks 12 - 36)-Results for ITT Data Analyses Set: Slope Estimates (SE)
- Figure 10B.: Results of Primary analysis #1 - Comparison of Slopes in the PC phase (weeks 12 - 36)-Results for ITT Data Analyses Set: Slope Estimates Difference and 95% CI
- Figure 11A.: Placebo Controlled Phase - Secondary Efficacy Endpoint Week 36: Adjusted Means of the Change from Baseline to LOV in Total UPDRS: Adjusted Means (SE)
- Figure 11B.: Placebo Controlled Phase - Secondary Efficacy Endpoint Week 36: Adjusted Means of the Change from Baseline to LOV in Total UPDRS: Adjusted Means Difference and 95% CI
- Figure 12.: Change in Total UPDRS - Rasagiline 1mg Early vs Delayed Start - ACTE: TVP-1012/500 (ADAGIO) Mean + - of Change in Total UPDRS Score ACTE Data Analysis Set
- Figure 13.: Change in Total UPDRS - Rasagiline 2mg Early vs Delayed Start - ACTE: TVP-1012/500 (ADAGIO) Mean + - of Change in Total UPDRS Score ACTE Data Analysis Set
- Figure 14A.: Results of Primary Analysis #2 - Comparison at Week 72 (end of the Active Phase): Adjusted Means (SE)
- Figure 14B.: Results of Primary Analysis #2 - Comparison at Week 72 (end of the Active Phase): Adjusted Mean Difference and 95% CI
- Figure 15A.: Results of Primary Analysis #3 - Non-Inferiority of Slopes in the Active Phase Weeks 48-72 - Results: Slope Estimates (SE)
- Figure 15B.: Results of Primary Analysis #3 - Non-Inferiority of Slopes in the Active Phase Weeks 48-72 - Results: Slope Estimates Difference and 95% CI
- Figure 16A.: PC Phase: % of patients Requiring Additional Anti-PD Therapy
- Figure 16B.: PC Phase: % of patients Requiring Additional Anti-PD Therapy: Odds Ratio and 95% CI
- Figure 17.: Time to additional anti-PD therapy in the PC Phase Kaplan Meier Curves and Cox Proportional Hazards Model Results
- Figure 18A.: PC Phase: Adjusted Means of the Change from Baseline to LOV in Part I of UPDRS version 4 (Non-Motor Aspects of Experiences of Daily Living): Adjusted Means (SE)
- Figure 18B.: PC Phase: Adjusted Means of the Change from Baseline to LOV in Part I of UPDRS version 4 (Non-Motor Aspects of Experiences of Daily Living): Adjusted Means Difference and 95% CI
- Figure 19A.: PC Phase: Adjusted Means of the Change from Baseline to LOV in Parkinson Fatigue Scale (PFS): Adjusted Means (SE)
- Figure 19B.: PC Phase: Adjusted Means of the Change from Baseline to LOV in Parkinson Fatigue Scale (PFS): Adjusted Means Difference and 95% CI

### Detailed Description of the Invention

The subject invention provides a method of reducing the rate of progression of Parkinson's disease symptoms in an early stage Parkinson's disease patient, the method comprising identifying an early stage Parkinson's disease patient, and periodically administering to the early stage Parkinson's disease patient so identified an amount of rasagiline, or a pharmaceutically acceptable salt of rasagiline effective to reduce the rate of progression of Parkinson's disease symptoms.

In an embodiment of this method, the patient is not receiving bromocriptine, benztropine, levodopa, ropinirole, pramipexole, rotigotine, cabergoline, entacapone, tolcapone, amantadine or selegiline.

In another embodiment of this method, the patient is not receiving any therapy for Parkinson's disease other than rasagiline or a pharmaceutically acceptable salt of rasagiline.

In yet another embodiment of this method, the Parkinson's disease symptoms are quantified by the Total Unified Parkinson's Disease Rating Scale (Total UPDRS) score, an increase in the Total UPDRS score represents progression of Parkinson's disease symptoms, and the increment of the increase in Total UPDRS score over a period of time represents the rate of progression of Parkinson's disease symptoms.

In yet another embodiment of this method, the period of time is 12, 24, or 36 weeks after initiation of administration of rasagiline or a pharmaceutically acceptable salt of rasagiline.

In yet another embodiment of this method, the rate of progression is an average Total UPDRS score increase of less than less than 0.15 units per week after the initial symptomatic effect period of the administration of rasagiline or a pharmaceutically acceptable salt of rasagiline. In addition, the period of time is from week 12 to week 36.

In yet another embodiment of this method, the rate of progression is an average Total UPDRS score increase of between 0.15 and 0.05 units per week after the initial symptomatic effect period of the administration of rasagiline or a pharmaceutically acceptable salt of rasagiline.

In yet another embodiment of this method, the rate of progression is an average Total UPDRS score increase of between 0.15 and 0.07 units per week after the initial symptomatic effect period of the administration of rasagiline or a pharmaceutically acceptable salt of rasagiline.

In yet another embodiment of this method, the rate of progression is an average Total UPDRS score increase of between 0.11 and 0.07 units per week after the initial symptomatic effect period of the administration of rasagiline or a pharmaceutically acceptable salt of rasagiline.

In yet another embodiment of this method, the period of time is 48, 54, 60, 66 or 72 weeks after initiation of administration of rasagiline or a pharmaceutically acceptable salt of rasagiline.

In yet another embodiment of this method, the amount of rasagiline administered is 1 mg per day.

In yet another embodiment of this method, the amount of rasagiline administered is 2 mg per day.

In yet another embodiment of this method, the pharmaceutically acceptable salt of rasagiline is rasagiline mesylate.

In yet another embodiment of this method, the early stage Parkinson's disease patient is a Stage I patient according to Hoehn and Yahr rating.

In yet another embodiment of this method, the early stage Parkinson's disease patient is a patient whose symptoms result in a UPDRS total score of less than 30; less than 25; less than 23; less than 21; or less than 20.

In yet another embodiment of this method, the early stage Parkinson's disease patient is a patient whose symptoms result in a UPDRS motor score of less than 17.5; less than 17; less than 16; less than 15; less than 14.5; or less than 14.

In yet another embodiment of this method, the early stage Parkinson's disease patient is a patient whose symptoms have been diagnosed to indicate Parkinson's disease within the prior 12; 11; 10; 9; 8; 7; 6; 5; 4; 3; 2; or 1 month(s).

The subject invention also provides a method of reducing the rate of progression of Parkinson's disease symptoms in a Parkinson's disease patient, the method comprising periodically administering to the Parkinson's disease patient for more than 52 weeks an amount of rasagiline, or a pharmaceutically acceptable salt of rasagiline effective to reduce the rate of progression of Parkinson's disease symptoms.

In an embodiment of this method, the patient is an early stage Parkinson's disease patient.

Additional embodiments of this method are described throughout the specification.

The subject invention further provides a method for delaying the need for symptomatic anti-Parkinsonian therapy in an early stage Parkinson's disease patient, comprising identifying a patient to be an early stage Parkinson's disease patient, and periodically administering to the patient so identified an amount of rasagiline or a pharmaceutically acceptable salt of rasagiline effective to delay the need for symptomatic anti-Parkinsonian therapy.

In an embodiment of this method, the delay in the need for symptomatic anti-Parkinsonian therapy is more than 34 weeks, more than 36 weeks, or more than 42 weeks after initiation of administration of rasagiline or a pharmaceutically acceptable salt of rasagiline.

Additional embodiments of this method are described throughout the specification.

The subject invention further provides a method for reducing the risk of a Parkinson's disease patient requiring symptomatic anti-Parkinsonian therapy, comprising periodically administering to the patient for 36 weeks an amount of rasagiline or a pharmaceutically acceptable salt of rasagiline effective to reduce the risk of requiring symptomatic anti-Parkinsonian therapy.

In an embodiment of this method, the risk is reduced by 40-60%. In particular, the risk is reduced by at least 50%.

In an embodiment of this method, the administration is for more than 52 weeks.

Additional embodiments of this method are described throughout the specification.

The subject invention further provides a method of reducing the functional decline of an early stage Parkinson's disease patient, comprising identifying a patient to be an early stage Parkinson's disease patient, and periodically administering to the patient so identified an amount of rasagiline or a pharmaceutically acceptable salt of rasagiline effective to reduce the functional decline.

In an embodiment of this method, functional decline of an early stage Parkinson's disease patient is quantified by the Total Unified Parkinson's Disease Rating Scale (Total UPDRS) score, an increase in the Total UPDRS score represents functional decline.

In another embodiment of this method, the increase in Total Unified Parkinson's Disease Rating Scale (Total UPDRS) score is less than 3.97 units or less than 3.35 units at 72 weeks after initiation of administration of rasagiline or a pharmaceutically acceptable salt of rasagiline.

Additional embodiments of this method are described throughout the specification.

The subject invention further provides a method of treating a patient exhibiting early signs of Parkinson's disease, comprising identifying a patient exhibiting early signs of Parkinson's disease, and periodically administering to the patient so identified an amount of rasagiline or a pharmaceutically acceptable salt of rasagiline effective to treat the patient.

Additional embodiments of this method are described throughout the specification.

The subject invention further provides a method of reducing fatigue in an early stage Parkinson's disease patient, comprising periodically administering to an early stage Parkinson's disease patient an amount of rasagiline or a pharmaceutically acceptable salt of rasagiline effective to reduce fatigue.

In an embodiment of this method, the Parkinson's Fatigue Scale is reduced by between 0.05 and 0.23 compared to a patient who is not being treated by rasagiline.

The subject invention further provides a method of reducing severity of non-motor symptoms in an early stage Parkinson's disease patient, comprising periodically administering to an early stage Parkinson's disease patient an amount of rasagiline or a pharmaceutically acceptable salt of rasagiline effective to reduce the severity of non-motor symptoms.

In an embodiment of this method, the non-motor symptoms are defined by UPDRS Version 4 part 1.

In another embodiment of this method, the change in UPDRS score as defined in Version 4 part 1 is at least 0.23 in comparison to a patient who did not undergo treatment with rasagiline.

The subject invention further provides a method of slowing clinical progression and treating symptoms of Parkinson's disease in a Parkinson's disease patient comprising periodically administering to the Parkinson's disease patient an amount of rasagiline or a pharmaceutically acceptable salt of rasagiline effective to slow clinical progression and treat the signs and symptoms of Parkinson's disease in the patient.

In an embodiment of this method, wherein the amount of rasagiline administered is 1 mg per day.

In another embodiment of this method, the patient is not receiving bromocriptine, benztropine, levodopa, ropinirole, pramipexole, rotigotine, cabergoline, entacapone, tolcapone, amantadine or selegiline.

In yet another embodiment of this method, the patient is not receiving any therapy for Parkinson's disease other than rasagiline or a pharmaceutically acceptable salt of rasagiline.

In yet another embodiment of this method, an average Total UPDRS score of the patient increases less than 0.15 units per week after the initial symptomatic effect period of the administration of rasagiline or a pharmaceutically acceptable salt of rasagiline.

In yet another embodiment of this method, an average Total UPDRS score of the patient increases between 0.15 and 0.05 units per week after the initial symptomatic effect period of the administration of rasagiline or a pharmaceutically acceptable salt of rasagiline.

In yet another embodiment of this method, an average Total UPDRS score of the patient increases between 0.15 and 0.07 units per week after the initial symptomatic effect period of the administration of rasagiline or a pharmaceutically acceptable salt of rasagiline.

In yet another embodiment of this method, an average Total UPDRS score of the patient increases between 0.11 and 0.07 units per week after the initial symptomatic effect period of the administration of rasagiline or a pharmaceutically acceptable salt of rasagiline.

In yet another embodiment of this method, the pharmaceutically acceptable salt of rasagiline is rasagiline mesylate.

In yet another embodiment of this method, the patient is an early stage Parkinson's disease patient.

Additional embodiments of this method are described throughout the specification.

In an embodiment of the above methods, the Parkinson's disease patient is a patient whose Total UPDRS score is more than 25.5.

In an embodiment of this method, wherein the amount of rasagiline administered is 2 mg per day.

In another embodiment of this method, an average Total UPDRS score of the patient increases less than 0.28 units per week after the initial symptomatic effect period of the administration of rasagiline or a pharmaceutically acceptable salt of rasagiline.

In yet another embodiment of this method, an average Total UPDRS score of the patient increases between 0.28 and 0.01 units per week after the initial symptomatic effect period of the administration of rasagiline or a pharmaceutically acceptable salt of rasagiline.

In yet another embodiment of this method, an average Total UPDRS score of the patient increases between 0.09 and 0.01 units per week after the initial symptomatic effect period of the administration of rasagiline or a pharmaceutically acceptable salt of rasagiline.

In yet another embodiment of this method, an average Total UPDRS score of the patient increases between 0.18 and 0.10 units per week after the initial symptomatic effect period of the administration of rasagiline or a pharmaceutically acceptable salt of rasagiline.

In an embodiment of this method, the increase in Total Unified Parkinson's Disease Rating Scale (Total UPDRS) score is less than 3.10 units or less than 2.61 units at 72 weeks after initiation of administration of rasagiline or a pharmaceutically acceptable salt of rasagiline.

In another embodiment of this method, wherein the amount of rasagiline administered is 1 mg per day.

Additional embodiments of this method are described throughout the specification.

The subject invention further provides rasagiline or a pharmaceutically acceptable salt of rasagiline for use in reducing the rate of progression of Parkinson's disease symptoms in an early stage Parkinson's disease patient.

Additional embodiments of this use are described throughout the specification.

The subject invention further provides rasagiline or a pharmaceutically acceptable salt of rasagiline for use in delaying the need for symptomatic anti-Parkinsonian therapy in an early stage Parkinson's disease patient.

Additional embodiments of this use are described throughout the specification.

The subject invention further provides rasagiline or a pharmaceutically acceptable salt of rasagiline for use in reducing the risk of an early stage Parkinson's disease patient requiring symptomatic anti-Parkinsonian therapy. Additional embodiments of this use are described throughout the specification.

The subject invention further provides rasagiline or a pharmaceutically acceptable salt of rasagiline for use in reducing the functional decline in an early stage Parkinson's disease patient.

Additional embodiments of this use are described throughout the specification.

The subject invention further provides rasagiline or a pharmaceutically acceptable salt of rasagiline for use in treating a patient exhibiting early signs of Parkinson's disease.

Additional embodiments of this use are described throughout the specification.

The subject invention further provides rasagiline or a pharmaceutically acceptable salt of rasagiline for use in reducing the fatigue in an early stage Parkinson's disease patient.

Additional embodiments of this use are described throughout the specification.

The subject invention further provides rasagiline or a pharmaceutically acceptable salt of rasagiline for use in reducing the severity of non-motor symptoms in an early stage Parkinson's disease patient.

Additional embodiments of this use are described throughout the specification.

The subject invention further provides rasagiline or a pharmaceutically acceptable salt of rasagiline for use in slowing clinical progression and treating symptoms of Parkinson's disease in a Parkinson's disease patient.

Additional embodiments of this use are described throughout the specification.

The subject invention yet further provides a pharmaceutical composition comprising a pharmaceutically effective amount of rasagiline or a pharmaceutically acceptable salt of rasagiline for use in reducing the rate of progression of Parkinson's disease symptoms in a Parkinson's disease patient.

Additional embodiments of this pharmaceutical composition are described throughout the specification.

The subject invention yet further provides a pharmaceutical composition comprising a pharmaceutically effective amount of rasagiline or a pharmaceutically acceptable salt of rasagiline for use in delaying the need for symptomatic anti-Parkinsonian therapy in an early stage Parkinson's disease patient.

Additional embodiments of this pharmaceutical composition are described throughout the specification.

The subject invention yet further provides a pharmaceutical composition comprising a pharmaceutically effective amount of rasagiline or a pharmaceutically acceptable salt of rasagiline for use in reducing the risk of a Parkinson's disease patient requiring symptomatic anti-Parkinsonian therapy.

Additional embodiments of this pharmaceutical composition are described throughout the specification.

The subject invention yet further provides a pharmaceutical composition comprising a pharmaceutically effective amount of rasagiline or a pharmaceutically acceptable salt of rasagiline for use in reducing the functional decline in a Parkinson's disease patient.

Additional embodiments of this pharmaceutical composition are described throughout the specification.

The subject invention yet further provides a pharmaceutical composition comprising a pharmaceutically effective amount of rasagiline or a pharmaceutically acceptable salt of rasagiline for use in treating a patient exhibiting early signs of Parkinson's disease.

Additional embodiments of this pharmaceutical composition are described throughout the specification.

The subject invention yet further provides a pharmaceutical composition comprising a pharmaceutically effective amount of rasagiline or a pharmaceutically acceptable salt of rasagiline for use in reducing the fatigue in an early stage Parkinson's disease patient.

Additional embodiments of this pharmaceutical composition are described throughout the specification.

The subject invention yet further provides a pharmaceutical composition comprising a pharmaceutically effective amount of rasagiline or a pharmaceutically acceptable salt of rasagiline for use in reducing the severity of non-motor symptoms in an early stage Parkinson's disease patient.

Additional embodiments of this use are described throughout the specification.

The subject invention yet further provides a pharmaceutical composition comprising a pharmaceutically effective amount of rasagiline or a pharmaceutically acceptable salt of rasagiline for use in slowing clinical progression and treating symptoms of Parkinson's disease in a Parkinson's disease patient.

Additional embodiments of this use are described throughout the specification.

In each of the embodiments disclosed herein, the numeric values and ranges of average and total UPDRS scores can also be as follows:
In an embodiment of the above methods, the rate of progression is an average Total UPDRS score increase of less than 0.129 per week after initiation of administration of rasagiline or a pharmaceutically acceptable salt of rasagiline. In particular, the rate of progression is an average Total UPDRS score increase of 0.066 per week after initiation of administration of rasagiline or a pharmaceutically acceptable salt of rasagiline. In addition, the period of time is from week 12 to week 36.

In another embodiment of the above methods, the rate of progression is an average Total UPDRS score increase of between 0.129 and 0.059 per week after initiation of administration of rasagiline or a pharmaceutically acceptable salt of rasagiline.

In yet another embodiment of the above methods, the rate of progression is an average Total UPDRS score increase of between 0.099 and 0.029 per week after initiation of administration of rasagiline or a pharmaceutically acceptable salt of rasagiline.

In yet another embodiment of the above methods, the rate of progression is an average Total UPDRS score increase of between 0.125 and 0.045 per week after initiation of administration of rasagiline or a pharmaceutically acceptable salt of rasagiline.

In an embodiment of the above methods, the rate of progression is an average Total UPDRS score increase of less than 0.125 per week after initiation of administration of rasagiline or a pharmaceutically acceptable salt of rasagiline.

In yet another embodiment of the above methods, the rate of progression is an average Total UPDRS score increase of between 0.108 and 0.078 per week after initiation of administration of rasagiline or a pharmaceutically acceptable salt of rasagiline.

In yet another embodiment of the above methods, the rate of progression is an average Total UPDRS score increase of between 0.082 and 0.050 per week after initiation of administration of rasagiline or a pharmaceutically acceptable salt of rasagiline.

In yet another embodiment of the above methods, the rate of progression is an average Total UPDRS score increase of between 0.101 and 0.069 per week after initiation of administration of rasagiline or a pharmaceutically acceptable salt of rasagiline.

In another embodiment of the above methods, the increase in Total Unified Parkinson's Disease Rating Scale (Total UPDRS) score is less than 3.0, less than 2.0, less than 1.7, between 1.3-3.0, or between 1.3-2.5 at 72 weeks after initiation of administration of rasagiline or a pharmaceutically acceptable salt of rasagiline.

Examples of pharmaceutically acceptable salts include, but are not limited to, mineral or organic acid salts of basic residues such as amines; alkali or organic salts of acidic residues such as carboxcylic acids. The salts can be made using an organic or inorganic acid. Such acid salts are bromides, sulfates, nitrates, phosphates, sulfonates, formates, tartrates, maleates, malates, citrates, benzoates, salicylates, ascorbates, tannate, and the like. Carboxylate salts are the alkaline earth metal salts, sodium, potassium or lithium.

Rasagiline can also be in its free base form. A process of manufacture of the crystalline rasagiline base is described in PCT publication WO 2008/076348, the contents of which are hereby incorporated by reference.

Rasagiline may be used alone to treat Parkinson's disease, or alternatively, it may be used as an adjunct to other Parkinson's disease treatment agents, such as any of bromocriptine, benztropine, levodopa, ropinirole, pramipexole, rotigotine, cabergoline, entacapone, tolcapone, amantidine and selegiline.

### Definitions

As used herein, "symptomatic anti-Parkinsonian therapy" includes any of bromocriptine, benztropine, levodopa, ropinirole, pramipexole, rotigotine, cabergoline, entacapone, tolcapone, amantidine and selegiline.

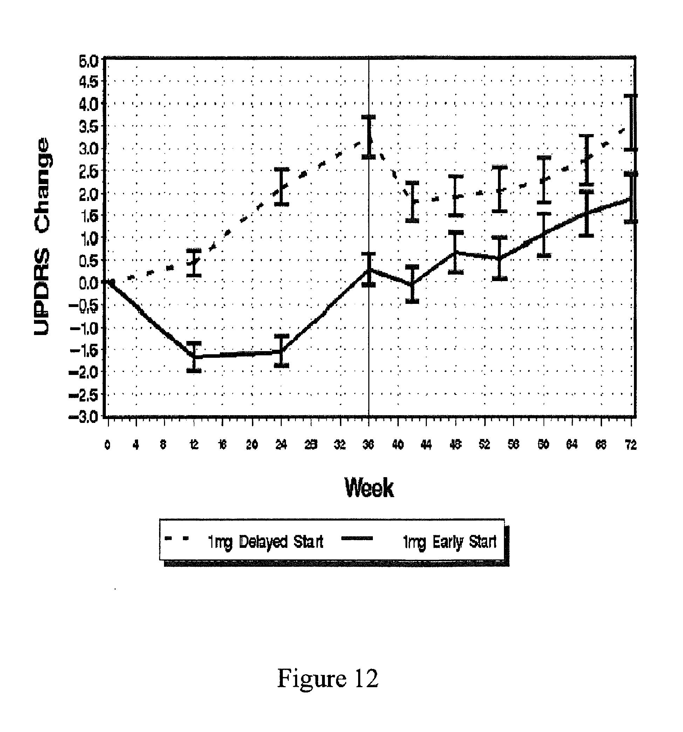

As used herein, "initial symptomatic effect period" is the period beginning immediately after a patient is administered rasagiline during which the total UPDRS score declines and ending when the total UPDRS score no longer declines, and in a preferred embodiment when the total UPDRS score starts to rise. For example, in the data represented in Figure 12, the initial symptomatic effect period is from week 0 to week 12 for the 1 mg early start group.

As used herein, "reducing the rate of progression of Parkinson's disease" means reducing the deterioration experienced by a PD patient, e.g. as quantified by UPDRS score, as compared to the deterioration experienced by a PD patient not receiving rasagiline over a period of time.

As used herein, "delaying the need for symptomatic anti-Parkinsonian therapy" means delaying the need for symptomatic anti-Parkinsonian therapy for a Parkinson's disease patient who receives rasagiline, as compared to a patient not receiving rasagiline.

As used herein, "functional decline" means the worsening of symptoms of a PD patient over time determinable using Total UPDRS score.

As used herein, "early signs of Parkinson's disease" includes one or more of the followings:
a) a resting 4- to 8-Hz pill-rolling tremor of one hand;
b) tremor which is maximal at rest, diminishes during movement, and is absent during sleep;
c) rigidity and slowing of movement (bradycardia), decreased movement (hypokinesia), and difficulty in initiating movement (akinesia);
d) the face becoming masklike, with mouth open and diminished blinking, which may be confused with depression;
e) the posture becoming stooped;
f) difficulty in initiating walking; the gait becoming shuffling with short steps, and the arms being held flexed to the waist so as to not swing with the stride;
g) steps occasionally inadvertently quickening, and the patient occasionally breaking into a run to keep from falling (festination);
h) tendency to fall forward (propulsion) or backward (retropropulsion) when the center of gravity is displaced, resulting from loss of postural reflexes;
i) Speech becoming hypophonic, with a characteristic monotonous, stuttering dysarthria;
j) Hypokinesia and impaired control of distal musculature resulting in micrographia and increased difficulty with daily living activities;
k) infrequent blinking and lack of facial expression;
l) decreased movement;
m) impaired postural reflexes; and/or
n) characteristic gait abnormality.

As used herein, stages of a PD patient is described by Hoehn and Yahr in following five distinct stages depending on the symptoms (Hoehn MM, Yahr MD, Parkinsonism: onset, progression and mortality. Neurology 1967, 17:427-42).
**Stage I**: (mild or early disease): Symptoms affect only one side of the body.
**Stage II:** Both sides of the body are affected, but posture remains normal.
**Stage III:** (moderate disease): Both sides of the body are affected, and there is mild imbalance during standing or walking. However, the person remains independent.
**Stage IV:** (advanced disease): Both sides of the body are affected, and there is disabling instability while standing or walking. The person in this stage requires substantial help.
**Stage V:** Severe, fully developed disease is present. The person is restricted to a bed or chair.

As used herein, an "early stage PD patient" is a PD patient at Stage I or II of the Parkinson's Disease as defined by Hoehn and Yahr, and who does not require symptomatic anti-Parkinsonian therapy. Preferably such PD patient does not require symptomatic treatment for at least the next 9 months. An early stage PD patient may be identified as such by performing relevant testing.

As used herein, to "slow clinical progression" of Parkinson's disease means to achieve the three hierarchal primary endpoints exemplified by the clinical trial described in detail herein. The first of the primary end points is a slower rate of Total UPDRS score increase during the period after the full symptomatic effect of rasagiline had been attained, e.g. after week 12 of rasagiline administration, as compared to a subject not receiving rasagiline. The second of the primary end points is a lower deterioration in Total UPDRS score after a period of time sufficient to eliminate variations caused by a delayed start of rasagiline treatment when compared to a subject whose rasagiline treatment was delayed. Such period of time necessarily being more than 52 weeks after initiation of rasagiline treatment; and preferably being at least 72 weeks after initiation of rasagiline administration. The third of the primary end points is a substantially similar rate of deterioration in Total UPDRS score, e.g. within 0.15 Total UPDRS units/week, during a period of time after the full symptomatic effect of a delayed start rasagiline administration have been attained, as compared to a subject whose rasagiline treatment was delayed. Such a period of time in the third primary endpoint is preferably 24 weeks or longer.

The Total UPDRS (Unified Parkinson's Disease Rating Scale) score represents the level or severity of Parkinson's disease symptoms. It is used for measuring the change from baseline in efficacy variables during the treatment. UPDRS consists of a three-part test. A total of 31 items are included in Parts I, II and III test. Each item receives a score ranging from 0 to 4 where 0 represents the absence of impairment and 4 represents the highest degree of impairment. The sum of Parts I, II and III at each study visit provides a Total UPDRS score. Part I is designed to rate mentation, behavior and mood (items 1-4). It is collected as historical information. Part II (items 5-17) is also historical information. Part III (items 18-31) is a motor examination at the time of a visit.

### Part I: Mentation, Behavior and Mood

Item 1. INTELLETUAL IMPAIRMENT
   - 0:: None.
   - 1:: Mild - Consistent forgetfulness with partial recollection of events and no other difficulties.
   - 2:: Moderate memory loss, with disorientation and moderate difficulty in handling complex problems. Mild but definitive impairment of function at home with need of occasional prompting.
   - 3:: Severe memory loss with disorientation for time and often to place. Severe impairment in handling problems.
   - 4:: Severe memory loss with orientation preserved to person only. Unable to make judgments or solve problems. Requires much help with personal care; cannot be left alone at all.
Item 2. THOUGHT DISORDERS (due to dementia or drug intoxication)
   - 0:: None.
   - 1:: Vivid dreaming.
   - 2:: Benign hallucinations with insight retained.
   - 3:: Occasional to frequent hallucinations or delusions; without insight; could interfere with daily activities.
   - 4:: Persistent hallucinations, delusions or florid psychosis. Not able to care for self.
Item 3. DEPRESSION
   - 0:: Not present.
   - 1:: Periods of sadness or guilt greater than normal. Never sustained for days or weeks.
   - 2:: Sustained depression (1 week or more)

   - 3:: Sustained depression with vegetative symptoms (insomnia, anorexia, weight loss, loss of interest).
   - 4:: Sustained depression with vegetative symptoms and suicidal, thoughts or intent.
Item 4. MOTIVATION/INITIATIVE
   - 0:: Normal.
   - 1:: Less assertive than usual; more passive.
   - 2:: Loss of initiative or disinterest in elective (non-routine) activities.
   - 3:: Loss of initiative or disinterest in day-to-day (routine) activities.
   - 4:: Withdrawn, complete loss of motivation.

### PART II: ACTIVITIES OF DAILY LIVING (score 0-4)

Item 5. SPEECH
   - 0:: Normal.
   - 1:: Mildly affected. No difficulty being understood.
   - 2:: Moderately affected. Sometimes asked to repeat statements.
   - 3:: Severely affected. Frequently asked to repeat statements.
   - 4:: Unintelligible most of the time.
Item 6. SALIVATION
   - 0:: Normal.
   - 1:: Slight but definite excess of saliva in mouth; may have nighttime drooling.
   - 2:: Moderately excessive of saliva; may have minimal drooling.
   - 3:: Marked excess of saliva with some drooling.
   - 4:: Marked drooling, requires constant tissue or handkerchief.
Item 7. SWALLOWING
   - 0:: Normal.

   - 1:: Rare choking.
   - 2:: Occasional choking.
   - 3:: Requires soft food.
   - 4:: Requires nasogastric tube or gastrotomy feeding.
Item 8. HANDWRITING
   - 0:: Normal.
   - 1:: Slightly slow or small.
   - 2:: Moderately slow or small; all words are legible.
   - 3:: Severely affected; not all words are legible.
   - 4:: The majority of words are not legible.
Item 9. CUTTING FOOD, HANDLING UTENSILS
   - 0:: Normal.
   - 1:: Somewhat slow, but no help needed.
   - 2:: Can cut most foods, although clumsy and slow; some help needed.
   - 3:: Food must be cut by someone, but can still feed slowly.
   - 4:: Needs to be fed.
Item 10. DRESSING
   - 0:: Normal.
   - 1:: Somewhat slow, but no help needed.
   - 2:: Occasional assistance with buttoning, getting arms in sleeves.
   - 3:: Considerable help required, but can do some things alone.
   - 4:: Helpless.
Item 11. HYGIENE
   - 0:: Normal.
   - 1:: Somewhat slow, but no help needed.
   - 2:: Needs help to shower or bathe, or very slow in hygienic care.
   - 3:: Requires assistance for washing, brushing teeth, combing hair, going to bathroom.
   - 4:: Foley catheter or other mechanical aids.
Item 12. TURNING IN BED AND ADJUSTING BED CLOTHES
   - 0:: Normal.
   - 1:: Somewhat slow and clumsy, but no help needed.
   - 2:: Can turn alone or adjust sheets, but with great difficulty.
   - 3:: Can initiate, but not turn or adjust sheets alone.
   - 4:: Helpless.
Item 13. FALLING (unrelated to freezing)
   - 0:: None.
   - 1:: Rare falling.
   - 2:: Occasionally falls, less than once per day.
   - 3:: Falls an average of once daily.
   - 4:: Falls more than once daily.
Item 14. FREEZING WHEN WALKING
   - 0:: None.
   - 1:: Rare freezing when walking; may have start-hesitation.
   - 2:: Occasional freezing when walking.
   - 3:: Frequent freezing. Occasionally falls from freezing.
   - 4:: Frequent falls from freezing.
Item 15. WALKING
   - 0:: Normal.
   - 1:: Mild difficulty. May not swing arms or may tend to drag leg.
   - 2:: Moderate difficulty, but requires little or no assistance.
   - 3:: Severe disturbance of walking, requiring assistance.
   - 4:: Cannot walk at all, even with assistance.
Item 16. TREMOR
   - 0:: Absent.
   - 1:: Slight and infrequently present.
   - 2:: Moderate; bothersome to patient.
   - 3:: Severe; interferes with many activities.
   - 4:: Marked; interferes with most activities.
Item 17. SENSORY COMPLAINTS RELATED TO PARKINSONISM
   - 0:: None.
   - 1:: Occasionally has numbness, tingling or mild aching.
   - 2:: Frequently has numbness, tingling or aching; not distressing.
   - 3:: Frequent painful sensations.
   - 4:: Excruciating pain.

### PART III: MOTOR EXAMINATION (score 0-4)

Item 18. SPEECH
   - 0:: Normal.
   - 1:: Slight loss of expression, diction and/or volume.
   - 2:: Monotone, slurred but understandable; moderately impaired.
   - 3:: Marked impairment, difficult to understand.
   - 4:: Unintelligible.
Item 19. FACIAL EXPRESSION
   - 0:: Normal.
   - 1:: Minimal hypomimia, could be normal "Poker Face".
   - 2:: Slight but definitely abnormal diminution of facial expression.
   - 3:: Moderate hypomania; lips parted some of the time.
   - 4:: Masked or fixed faces with severe or complete loss of facial expression; lips parted 1/4 inch or more.
Item 20. TREMOR AT REST
   a) Face, lips and chin
   b) Right hand
   c) Left hand
   d) Right foot
   e) Left foot
      - 0:: Absent.
      - 1:: Slight and infrequently present.

      - 2:: Mild in amplitude and persistent; or moderate in amplitude, but only intermittently present.
      - 3:: Moderate in amplitude and present most of the time.
      - 4:: Marked in amplitude and present most of the time.
Item 21. ACTION OR POSTURAL TREMOR OF HANDS
   - 0:: Absent.
   - 1:: Slight; present with action.
   - 2:: Moderate in amplitude, present with action.
   - 3:: Moderate in amplitude with posture holding as well as action.
   - 4:: Marked in amplitude; interfere with feeding.
Item 22. RIGIDITY (Judged on passive movement of major
   joints with subject relaxed in sitting position. Cogwheeling to be ignored)
   a) neck
   b) right upper extremities
   c) left upper extremities
   d) right lower extremities
   e) left lower extremities
      - 0:: Absent.
      - 1:: Slight or detectable only when activated by mirror or other movements.
      - 2:: Mild or moderate.
      - 3:: Marked, but full range of motion easily achieved.
      - 4:: Severe, range of motion achieved with difficulty.
Item 23. FINGER TAPS (Subject taps thumb with index
   finger in rapid succession with widest amplitude possible, each hand separately)
   a) Right hand
   b) Left hand
      - 0:: Normal > 15/5 sec.
      - 1:: Mild slowing and/or reduction in amplitude (11-14.5 sec).
      - 2:: Moderately impaired. Definite and early fatiguing. May have occasional arrests in movement (7-10/5 sec).
      - 3:: Severely impaired. Frequent hesitation in initiating movements or arrests in ongoing movement (3-6/5 sec).
      - 4:: Can barely perform the task (0-2/5 sec).
Item 24. HAND MOVEMENT (Subject opens and closes hands
   in rapid succession with widest amplitude possible, each hand separately)
   a) Right hand
   b) Left hand

   - 0:: Normal.
   - 1:: Mild slowing and/or reduction in amplitude.
   - 2:: Moderately impaired. Definite and early fatiguing. May have occasional arrests in movements.
   - 3:: Severely impaired. Frequent hesitation in initiating movements or arrests in ongoing movement.
   - 4:: Can barely perform the task.
Item 25. RAPID ALTERNATING MOVEMENTS OF HANDS (Pronation, supination movements of hands, vertically or horizontally with as large an amplitude as possible, both hands simultaneously)
   - 0:: Normal.
   - 1:: Mild slowing and/or reduction in amplitude.
   - 2:: Moderately impaired. Definite and early fatiguing. May have occasional arrests in movement.
   - 3:: Severely impaired. Frequent hesitation in initiating movements or arrests in ongoing movement.
   - 4:: Can barely perform the task.
Item 26. LEG AGILITY (Subject taps heel on ground in rapid succession, picking up entire leg. Amplitude should be about 3 inches)
   - 0:: Normal.

   - 1:: Mild slowing and/or reduction in amplitude.
   - 2:: Moderately impaired. Definite and early fatiguing. May have occasional arrest in movement.
   - 3:: Severely impaired. Frequent hesitation in initiating movements or arrests in ongoing movement.
   - 4:: Can barely perform the task.
Item 27. ARISING FROM CHAIR (Subject attempts to arise from a straight-back wood or metal chair with arms folded across)
   - 0:: Normal.
   - 1:: Slow, or may need more than one attempt.
   - 2:: Pushes self up from arms of seat.
   - 3:: Tends to fall back and may have to try more than one time, but can get up without help.
   - 4:: Unable to arise without help.
Item 28. POSTURE
   - 0:: Normal erect.
   - 1:: Not quite erect, slightly stooped posture; could be normal for older person.
   - 2:: Moderately stooped posture, definitely abnormal, can be slightly leaning to one side.
   - 3:: Severely stooped posture with kyphosis; can be moderately leaning to one side.
   - 4:: Marked flexion with extreme abnormality of posture.
Item 29. GAIT
   - 0:: Normal.
   - 1:: Walks slowly, may shuffle with short steps, but no festination or propulsion.
   - 2:: Walks with difficulty, but requires little or no assistance; may have some festination, short steps, or propulsion.
   - 3:: Severe disturbance, of gait requiring assistance.
   - 4:: Cannot walk at all, even with assistance.
Item 30. POSTURAL STABILITY (Response to sudden posterior displacement)
   - 0:: Normal.
   - 1:: Retropulsion, but recovers unaided.
   - 2:: Absence of postural response; would fall if not caught by examiner.
   - 3:: Very unstable, tends to lose balance spontaneously.
   - 4:: Unable to stand without assistance.
Item 31. BODY BRADYKINESIA AND HYPOKINESIA (Combining slowness, hesitancy, decreased arm swing, small amplitudes and poverty of movement in general)
   - 0:: None.
   - 1:: Minimal slowness, giving movement a deliberate character; could be normal for some person. Possibly reduced amplitude.
   - 2:: Mild degree of slowness and poverty of movement which is definitely abnormal. Alternatively, some reduced amplitude.
   - 3:: Moderate slowness, poverty or small amplitude of movement.
   - 4:: Marked slowness, poverty or small amplitude of movement.

### Discussions

Numerous agents have been considered to have putative neuroprotective effects based on laboratory research, but none has been established to provide a neuroprotective effect in PD despite clinical trials with positive outcome measures (Schapira AH, Olanow CW. Neuroprotection in Parkinson disease: mysteries, myths, and misconceptions. JAMA. 2004, 291:358-364). One of the major limitations in defining a neuroprotective therapy has been the lack of an outcome measure that accurately reflects the underlying disease state and is not confounded by symptomatic or pharmacologic effects of the study intervention. The MAO-B inhibitor rasagiline (Azilect®) has been demonstrated to be an anti-apoptotic agent that has neuroprotective effects in laboratory models (Olanow GW. Rationale for considering that propargylamines might be neuroprotective in Parkinson's disease. Neurology. 2006, 66(10 Suppl 4): S69-79). However, rasagiline has been demonstrated to provide statistically significant antiparkinsonian benefits when used as monotherapy in early PD (Parkinson Study Group. A controlled trial of rasagiline in early Parkinson disease: the TEMPO Study. Arch Neurol 2002, 59:1937-1943) or as an adjunct to levodopa in PD patients experiencing motor fluctuations (Parkinson Study Group. A randomized placebo-controlled trial of rasagiline in levodopa-treated patients with Parkinson disease and motor fluctuations: the PRESTO study. Arch Neurol 2005, 62:241-248; Rascol O, Brooks DJ, Melamed E, et al. Rasagiline as an adjunct to levodopa in patients with Parkinson's disease and motor fluctuations (LARGO, Lasting effect in Adjunct therapy with Rasagiline Given Once daily, study): a randomised, double-blind, parallel-group trial. Lancet. 2005-365:947-54), and thus might be expected to introduce a symptomatic confound into a neuroprotective study using outcome measures that have been employed to date.

A clinical study ("TEMPO study") for the development of rasagiline as a symptomatic anti-PD agent has been completed. The first 6-month double-blind, randomized placebo-controlled stage of the TEMPO study demonstrated that rasagiline is efficacious as monotherapy in the treatment of PD patients (Parkinson Study Group. A Controlled Trial of Rasagiline in Early Parkinson Disease. The TEMPO Study. Arch Neurol, December 2002, Vol 59: 1937-1943). TEMPO employed a randomized start design, and TEMPO results can be viewed to suggest that subjects treated with 1 and 2 mg/d rasagiline for one year show less functional decline than subjects whose treatment is delayed for 6 months. This, however, cannot be conclusively determined for the 1mg dose, as the study included only 3 groups and at the second phase all subjects were treated by 2mg rasagiline. Because all subjects were receiving rasagiline in the second stage of the study and the symptomatic effects of the drug were presumably balanced at the last examination, it seems that the differences in performance observed at the final visit may be due to rasagiline's disease modifying effect. Since this was not the primary objective of the TEMPO study, these results warranted an additional, definitive clinical trial.

For a therapy to be effective in modifying PD, neuroprotection must be introduced as early in the course of disease as possible. This is due to the reason that by the time a diagnosis of PD is made, 50% to 80% of nigral cell loss has usually already occurred (Simpins N, Jankovic J. Neuroprotection in Parkinson Disease. Arch Intern Med, July 28, 2003, Vol 163: 1650-1654). Therefore, unlike the TEMPO study discussed above, the current study focused only on early stage PD patients.

Furthermore, unlike the TEMPO study, the current study was designed to properly investigate and confirm the suggestion of disease modifying effect by starting with 1100 subjects in order to provide 87% power to detect a difference of greater than or equal to 1.8 UPDRS points between early- and delayed-start groups in mean change from baseline to weeks 48-72 with alpha=0.05 and 15% dropout. As discussed in the Study Design section, the primary hierarchal endpoints based on Total UPDRS score were set to confirm a disease modifying effect. The first endpoint compared slope estimates (change in UPDRS units/week) between the rasagiline (1 or 2 mg/day) and placebo groups from weeks 12- 36. This determines if there was a difference in the rate of progression of UPDRS score between each rasagiline group and placebo after week 12, when it was assumed that the full symptomatic effect of rasagiline had been established. A disease modifying agent would be expected to slow the rate of progression compared to placebo.

The second endpoint compared estimated change in total UPDRS score between baseline and week 72 in the rasagiline (1 or 2 mg/day) early-start and delayed-start groups. This determines if benefits observed in the early-start group at the end of phase I were still present at the end of the study when subjects in early- and delayed-start groups were receiving the same treatment.

The third endpoint tested for non-inferiority of UPDRS slope estimates between weeks 48-72 in the early- and delayed-start groups. A non-inferiority margin of 0.15 UPDRS units/week was pre-specified. This endpoint was designed to determine if the difference between groups was enduring (as would be expected with a disease-modifying effect) and not diminishing (as would be expected with a symptomatic agent). For each dose, all three endpoints have to be met to declare the study positive.

**Baseline characteristics of patients enrolled in ADAGIO vs. TEMPO**

| | **ADAGIO** | **TEMPO** |
|---|---|---|
| Patients randomazed (n) | 1176 | 404 |
| Male patients (n, %) | 718 (61.1%) | 257 (63.6%) |
| Age, years (mean, SD) | 62.2 (9.6) | 60.8 (10.8) |
| PD duration, months (mean, SD) | 4.5 (4.6) | 12.12 (13.2) |
| UPDRS total score (mean, SD) | 20.4 (8.5) | 25.03 (10.84) |
| UPDRS motor score (mean, SD) | 14.2 (6.4) | 17.8 (8.43) |
| Modified Hoehn & Yahr (mean, SD) | 1.5 (0.5) | 1.9 (0.5) |

This invention is illustrated in the following diagram where introduction of study intervention to patients in the placebo group (delayed start) did not provide benefit equal to that observed in subjects in the early start group and this difference persisted over several study visits. Thus, the delayed start group did not catch up to the early start group and the slopes of the rate of progression did not converge (i.e. lines remain parallel). This result cannot be readily explained by a symptomatic benefit and is consistent with the study intervention having a neuroprotective or disease modifying effect.

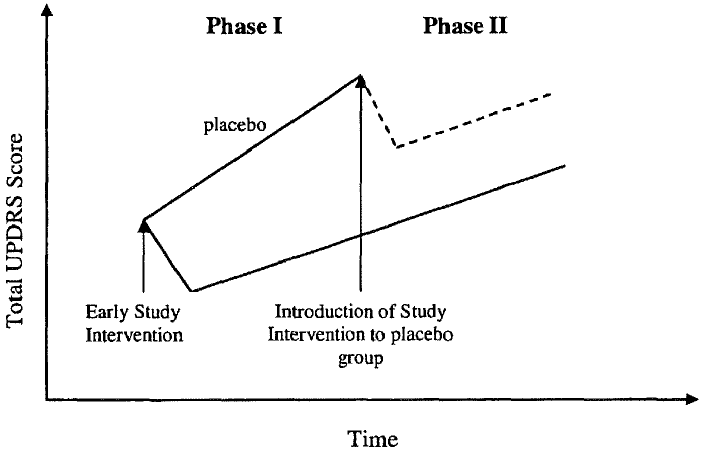

### Examples

### Clinical Trial - Evaluating Disease Modifying Effect in Early Stage PD Patients.

A prospective, multi-center, placebo-controlled, double-blind clinical trial ("ADAGIO") was conducted to show that rasagiline has a disease modifying effect in Early Stage PD patients.

### Study Design

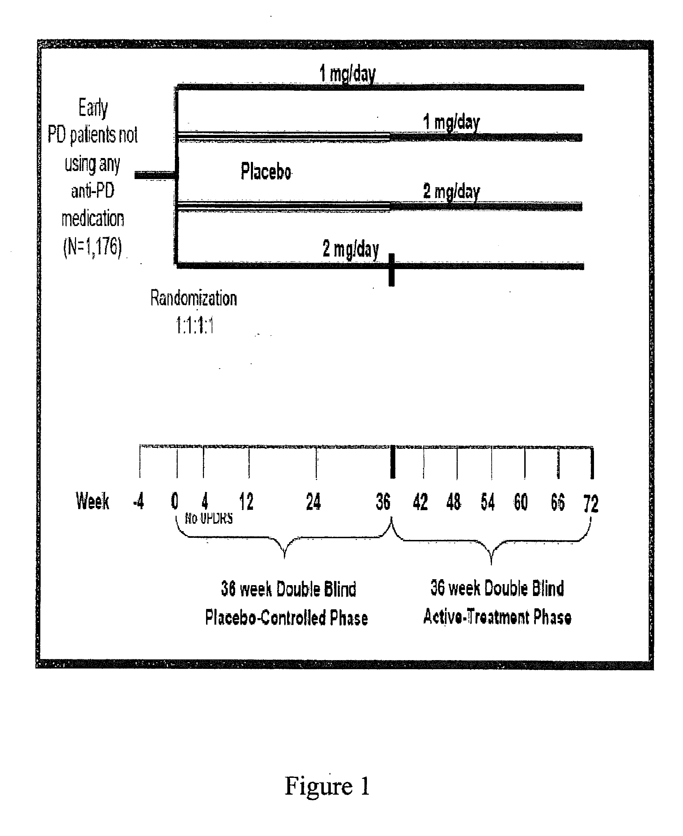

A randomized delayed start design was chosen for the study, which was comprised of 2 phases: Phase I - a 36-week double-blind, placebo-controlled phase, and Phase II - a 36-week double-blind, active-treatment phase. As illustrated in Figure 1 below, after being found eligible to participate in the study, subjects were allocated in a 1:1:1:1 ratio into one of the following four treatment groups based on a randomization scheme with blocks stratified by centers:
Group 1. 1 mg/day rasagiline during Phase I and Phase II;
Group 2. 2 mg/day rasagiline during Phase I and Phase II;
Group 3. placebo during Phase I followed by 1 mg/day
   rasagiline during Phase II; and
Group 4. placebo during Phase I followed by 2 mg/day
   rasagiline during Phase II.

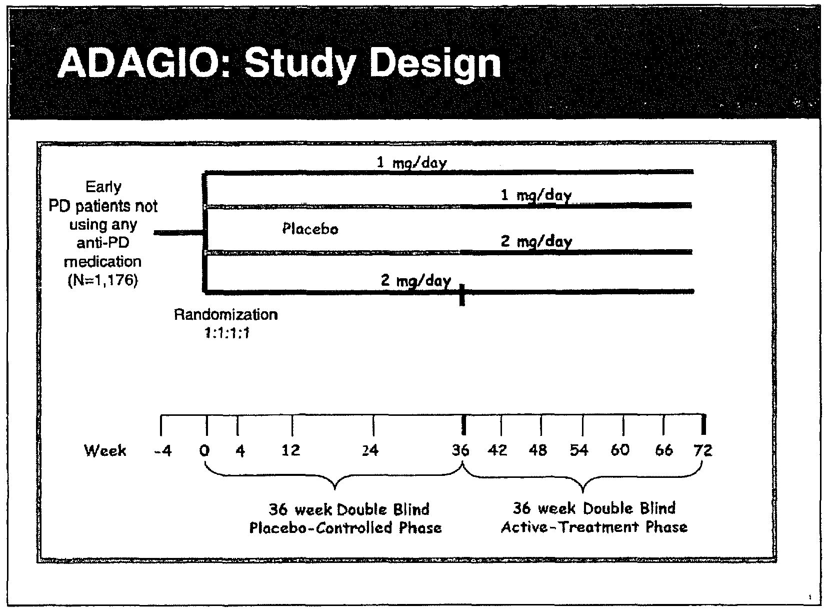

Thus, 'early-start' patients receive 72 weeks of treatment with rasagiline (1 or 2 mg once daily) and 'delayed-start' patients receive 36 weeks of placebo followed by 36 weeks of rasagiline (1 or 2 mg once daily). The 36-week duration of Phase I, the placebo-controlled period, was estimated to be long enough to establish a difference between active treatment and placebo, and a time period during which the average patient could remain on placebo without needing symptomatic therapy. If subjects in either treatment group required additional anti-parkinsonian medication during the placebo-controlled stage of the trial, they could proceed directly to Phase II. Once in Phase II, no additional anti-PD therapy is permitted. If the patient requires additional medication in this stage they are discontinued from the study.

### Phase I: Double-Blind Placebo-Controlled

Based on the above randomization scheme, subjects received either 1 mg/day or 2 mg/day rasagiline or placebo for 36 weeks during this stage.

### Phase II: Double-Blind Active-Treatment

Based on the above randomization scheme, all subjects received active treatment (either 1 mg or 2 mg rasagiline) during this stage according to their original randomization allocation. Thus, subjects who had received 1 mg rasagiline during Phase I continued to receive 1 mg during Phase II, subjects who had received 2 mg rasagiline during Phase I continued to receive 2 mg during Phase II, and subjects who had received placebo during Phase I received either 1 mg or 2 mg rasagiline during Phase II. These latter subjects were classified as "delayed 1 mg" and "delayed 2 mg" patients respectively to differentiate them from subjects who received active treatment for the entire duration of the study - the early-start subjects.

### Synopsis

A. Protocol Number
   TVP 1012/500 (ADAGIO)
B. Protocol Title
   A Multi-Center, Double-Blind, Randomized Start, Placebo-Controlled, Parallel-Group Study to Assess Rasagiline as a Disease Modifying Therapy in Early Parkinson's Disease Subjects
C. Clinical Trial Phase
   III B
D. Investigational Medicinal Product (IMP) & Dosage
   One tablet of 1 mg or 2 mg rasagiline, or matching placebo.
E. Study Duration
   Screening: up to approximately 4 weeks Study drug treatment: 36-week placebo-controlled phase followed by 36-week active-treatment phase.
F. Study Population
   Very early-phase subjects with idiopathic PD who have not developed sufficient disability to require any anti-PD therapy.
G. Study Objective
   To assess rasagiline as a disease modifying therapy in early PD.
H. Study Design
   This study will be comprised of 2 phases: Phase I - a 36-week double-blind, placebo-controlled phase, and Phase II - a 36-week double-blind, active-treatment phase. After being found eligible to participate in the study, subjects will be allocated in a 1:1:1:1 ratio into one of the following four treatment groups based on a randomization scheme with blocks stratified by centers:
   1. 1 mg/day rasagiline during Phase I and Phase
      II (1 mg early start)
   2. 2 mg/day rasagiline during Phase I and Phase II (2 mg early start)
   3. placebo during Phase I followed by 1 mg/day rasagiline during Phase II (1 mg delayed start)
   4. placebo during Phase I followed by 2 mg/day rasagiline during Phase II (2 mg delayed start)

### I. Phase I: Double-Blind Placebo-Controlled

Based on the above randomization scheme, subjects will receive either 1 mg/day or 2 mg/day rasagiline or placebo for 36 weeks during this phase.

If, at any stage during the placebo-controlled phase the investigator determines that a subject needs additional anti-PD therapy, the subject will proceed to Phase II of the study. The investigator, aided by a questionnaire, will give careful consideration to the issue of a subject's need for additional anti-PD therapy. Scheduled in-clinic visits will be conducted at baseline and at weeks 4, 12, 24 and 36. Therefore, altogether there will be 5 scheduled visits during this phase. Unscheduled visits may be conducted at any time to assess a subject's need for additional anti-PD therapy, for safety reasons or for any other reason.

### II. Phase II: Double-Blind Active-Treatment

Based on the above randomization scheme, all subjects will receive active treatment (either 1 mg or 2 mg rasagiline per day) for 36 weeks during this phase according to their original randomization allocation. Thus, subjects who receive 1 mg rasagiline during Phase I will continue to receive 1 mg during Phase II, subjects who receive 2 mg rasagiline during Phase I will continue to receive 2 mg during Phase II, and subjects who receive placebo during Phase I will receive either 1 mg or 2 mg rasagiline during Phase II. The study blind will be maintained. No additional anti-PD therapy will be permitted during this phase. This applies to both the subjects who enter Phase II after completing the full 36-week duration of Phase I and to subjects who are switched from Phase I to Phase II because of a need of additional anti-PD therapy. If, the investigator determines that a subject needs additional anti-PD therapy during Phase II, the subject will be prematurely withdrawn from the study. The investigator, aided by a questionnaire, will give careful consideration to the issue of a subject's need for additional anti-PD therapy. Scheduled in-clinic visits will be conducted every 6 weeks. Therefore, altogether there will be 6 visits during this phase - at weeks 42, 48, 54, 60, 66 and 72. Unscheduled visits may be conducted at any time to assess a subject's need for additional anti-PD therapy, for safety reasons or for any other reason.

### I. Number of Subjects

Approximately 1100 randomized subjects from 130 study sites.

### J. Inclusion/Exclusion Criteria

### I. Inclusion Criteria:

Subjects must meet all the inclusion criteria to be eligible:
1. Men and women with idiopathic Parkinson's disease whose diagnosis is confirmed at screening by the presence of at least two of the cardinal signs (resting tremor, bradykinesia, rigidity), without any other known or suspected cause of parkinsonism. If tremor is not present, subjects must have unilateral onset and persistent asymmetry.
2. Subjects with a diagnosis of early idiopathic PD of less than 1½ years duration from time of documented diagnosis.
3. Subjects whose clinical condition at the time of study enrollment does not require any anti-PD treatment and, to the best of the investigator's judgment, will not require for the next 9 months.
4. Willing and able to give informed consent.

### II. Exclusion Criteria:

Any of the following will exclude the subject from the study:
1. Subjects younger than 30 or older than 80 years of age.
2. Subjects with a loss of postural reflexes.
3. Subjects with a UPDRS Tremor score of 3 or greater in any limb.
4. Subjects with a Hoehn & Yahr Stage of III or greater at screening.
5. Subjects with freezing while walking.
6. Subjects with any one of the following features that tend to exclude PD as the cause of Parkinsonism:
   a. History of repeated strokes with stepwise progression of Parkinsonian features
   b. History of repeated head injury or history of definite encephalitis
   c. Sustained remission
   d. Supranuclear gaze palsy
   e. Cerebellar signs
   f. Early severe autonomic involvement
   g. Babinski's sign
   h. Presence of a cerebral tumour or communicating hydrocephalus
   i. MPTP exposure
   j. Oculogyric crises
7. Subjects who have had any previous use of rasagiline or selegiline,
8. Subjects having used any other anti-PD medication (including anticholinergics) on a chronic (for more than 3 weeks) basis at any time prior to baseline.
9. Subjects having used any other anti-PD medication (including anticholinergics) for less than 3 weeks during the 3 month period prior to baseline, (not including a single L-Dopa dose as part of L-Dopa test).
10. Subjects having used any other anti-PD medication (including anticholinergics) for less than 3 weeks prior to the 3 month period preceding baseline whose anti-PD medication is intentionally ceased in order for the subject to enter the study.
11. Subjects who, based on the investigator's judgment, have a clinically significant or unstable medical or surgical condition that may preclude safe and complete study participation. Such conditions may include cardiovascular, vascular disease, pulmonary, hepatic impairment (Child-Pugh Score >5), renal, or metabolic diseases or malignancies as determined by medical history, physical examination, laboratory tests, chest x-ray, or ECG.
12. Hypertensive subjects whose BP, in the investigator's opinion, is not well controlled according to the subject's medical record or as observed during the week of home BP recording prior to baseline.
13. Subjects diagnosed with melanoma based on the screening dermatologic examination, or with a history of melanoma. Subjects with suspicious lesions at baseline who do not undergo biopsy.
14. Subjects with significant cognitive impairment as defined by MMSE score < 26.
15. Subjects with clinically significant psychiatric illness, including major depression [Beck Depression Inventory (short form) >15].
16. Subjects with a history of alcohol or substance abuse within the past 2 years.
17. Subjects who have taken any experimental medications within 60 days prior to baseline.
18. Subjects who have used coenzyme Q10 (in daily doses > 300 mg) within 120 days prior to baseline.
19. Subjects who have used sympathomimetics (including over-the-counter remedies - nasal or oral), dextromethorphan, pethidine or St. John's Wort within the 7 days prior to baseline.
20. Subjects who have used antidepressants, including selective serotonin reuptake inhibitors, tricyclic and tetracyclic antidepressants (except: amitriptyline < 50 mg/daily, or trazodone < 100 mg/daily, or citalopram < 20 mg/daily, or sertraline < 100 mg/daily or paroxetine < 30 mg/daily, or escitalopram < 10 mg/daily used as single drug therapy) within 42 days prior to baseline.
21. Subjects who have used ciprofloxacin, a potent CYP 1A2 inhibitor within 7 days prior to baseline.
22. Subjects who have used MAO inhibitors including reserpine or methyldopa within the three months prior to baseline, or treatment with an anti-emetic or antipsychotic medication with central dopamine antagonist activity within the six months prior to baseline.
23. Women who are not postmenopausal, surgically sterilized, or using adequate birth control [oral birth control pills, IUD, or a long acting injectable form of contraception; barrier methods alone (i.e., condom) are not sufficient]. Women of childbearing potential without a negative pregnancy test (serum beta-HCG) at screening. Nursing women.

### K. Outcome Measures

### I. Primary Efficacy Endpoint

The change from baseline in Total UPDRS scores.

### II. Secondary Efficacy Endpoints

The change from baseline to Last Observed Values (LOV) in the placebo-controlled phase in Total UPDRS scores.

### III. Additional Efficacy Endpoints

1. Number (%) of subjects who need additional anti-PD therapy
2. Time to need of additional anti-PD therapy
3. Change from baseline to LOV in placebo-controlled phase in Part I of revised UPDRS (version 4)

### IV. Sub-study

1. Change from baseline to LOV in placebo-controlled phase in Parkinson Fatigue scale
2. Change from baseline in Subject Reported Outcomes (US only)
3. A blood sample for pharmacogenetic analysis will be collected from all subjects at baseline. If not performed at baseline, can be performed at any other visit. Participation in this sub-study is voluntary pending IRB/EC approval and subject's authorization on a separate informed consent form.

### IV. Safety and Tolerability Endpoints

Safety and tolerability will be evaluated with reference to the following:
1. Tolerability
   a. Number of subjects (%) who discontinue the study
   b. Number of subjects (%) who discontinue the study due to AEs
2. Safety Measures
   a. AE incidence
   b. Safety laboratory values
   c. Vital signs
   d. Home blood pressure monitoring
   e. ECG
   f. Physical and neurological examination
   g. Skin examination

### L. Statistical Methods

### I. Sample Size Rationale

A total of 935 subjects entering the active treatment phase (about 1100 subjects randomized, assuming 15% dropouts), will provide:
87% power for detection (at 5% significance level) of a statistical significant difference of a mean of 1.8 UPDRS points or more in the change from baseline in Total UPDRS during the active-treatment phase between the two treatment groups.
99% power to detect (at 2.5% significance level due to adjustment for multiple comparisons, and non-inferiority threshold of 0.15 UPDRS points per week) non-inferiority between the slopes of the rasagiline 1 mg (2 mg) early-start group as compared to rasagiline 1 tng (2 mg) delayed-start group.

### II. Primary Efficacy Endpoint

The primary efficacy endpoint for this trial will be the change from baseline in Total UPDRS score.

### III. Principal Statistical Analysis and Multiple Comparison Adjustment

The principal efficacy analysis will consist of three hierarchical statistical hypothesis testing that will be applied on the primary efficacy endpoint.

The Hochberg's Step-Up Bonferroni method for multiple comparisons between treatment groups (2 comparisons: early-start group compared to delayed-start group for Rasagiline 1mg and 2mg), in combination with the hierarchical method for the three hypotheses testing, will be used to maintain the experiment-wise type I error of 5% according to the following procedure:
If the first null hypothesis comparing the early-start group to the delayed-start group will be rejected for both rasagiline doses at an alpha level of 5% then no adjustment to the alpha level will be performed and both comparisons will be declared as statistically significant.
If the first null hypothesis will not be rejected for one of the doses at an alpha level of 5%, then the other dose will be tested using an alpha level of 5%/2 = 2.5%.

Each statistically significant dose, as determined by the test of hypothesis #1, will be further tested for hypothesis #2 using an alpha level of 5% and the Hochberg's Step-Up Bonferroni method as described for hypothesis #1 above.

Each statistically significant dose, as determined by the test of hypothesis #2, will be further tested for hypothesis #3 using an alpha level of 5% and the Hochberg's Step-Up Bonferroni method as described for hypothesis #1 above.

Following are the three hypothesis and the statistical models of the principal efficacy analysis:
1. Hypothesis #1: Slopes Superiority of
   Rasagiline over Placebo in the PC Phase
   H₀: Slope_{(Rasagiline)} - Slope_{(placebo)} = 0
   H_{A}: Slope_{(Rasagiline)} - Slope_{(placebo)} ≠ 0

Where slope is the model estimate of the change from baseline in total UPDRS per week. In this analysis, all available post baseline observations in the PC phase of the trial will be analyzed (ITT data analysis set, weeks 12, 24 and 36).

The placebo groups for rasagiline 1mg and 2 mg will be combined to one placebo group. The statistical model will be a Repeated Measures Mixed Linear Model with random intercept and slope (SAS® MIXED procedure with RANDOM sub-command). The model will include the following fixed effects: treatment group, continuous week in trial by treatment interaction, center and baseline Total UPDRS score. The individual subject intercept and the week effects will also be included in the model as random effects. The unstructured covariance matrix between the intercept and slopes estimates will be used.

Two comparisons will be derived from the model: slope difference of rasagiline 1mg group from the placebo group and slope difference of rasagiline 2mg group from the placebo group.
2. Hypothesis #2: Superiority of Early over Delayed Start at Week 72
   H₀: LSM_{(Early Start Group at Week 72)} -
   LSM_{(Delayed Start Group at Week 72)} = 0
   H_{A}: LSM_{(Early Start Group at Week 72)} -
   LSM_{(Delayed Start Group at Week 72)} ≠ 0
   Where LSM is Least Square Means

In this analysis, observations of all subjects entering the active phase with at least 24 weeks of treatment during the PC phase and at least one available Total UPDRS measurement during the active-treatment phase from weeks 48, 54, 60, 66 or 72, will be analyzed (ACTE data analysis set).

The statistical model will be a Repeated Measures model (SAS® MIXED procedure with REPEATED subcommand). The model will include the following fixed effects: categorical week in trial by treatment interaction, center, and baseline Total UPDRS score. The unstructured covariance matrix for repeated observations within subjects will be used. In case that the model will not converge, a simpler covariance structure with less parameters will be used, e.g. Heterogeneous Autoregressive(1) (ARH(1)) or Autoregressive(1) (AR(1)).

The LSM at week 72 of the change from baseline in Total UPDRS will be compared between the rasagiline 1mg early-start group and the rasagiline 1mg delayed-start group and between the rasagiline 2mg early-start group and the rasagiline 2mg delayed-start group.
3. Hypothesis #3: Slopes Non-Inferiority of Early Start over Delayed Start in the Active Phase
   H₀: Slope_{(Early Start Group)} - Slope_{(Delayed Start Group)} > 0.15
   H_{A}: Slope_{(Early Start Group)} - Slope_{(Delayed Start Group)} ≤ 0.15

In this analysis, observations of all subjects entering the active phase with at least 24 weeks of treatment during the PC phase and at least, one available Total UPDRS measurement during the active-treatment phase from weeks 48, 54, 60, 66 or 72, will be analyzed (ACTE data analysis set).

The statistical model will be a Repeated Measures Mixed Linear Model with random intercept and slope (SAS® MIXED procedure with RANDOM sub-command). The model will include the following fixed effects: treatment group, continuous week in trial by treatment interaction, center and baseline Total UPDRS score. The individual subject intercept and the week effects will also be included in the model as random effects. The unstructured covariance matrix between the intercept and slopes estimates will be used.

Non-inferiority test for the difference in slopes between the treatment groups will be performed.

The one sided 95% Confidence Interval (CI) will be calculated for the difference between the slopes of the rasagiline 1mg early-start group and the rasagiline 1mg delayed-start group and between the slopes of the rasagiline 2mg early-start group and the rasagiline 2mg delayed-start rasagiline group. The inferiority null hypothesis of the early-start group slope over the delayed-start group slope will be rejected, if the upper limit of the one sided 95% CI for the difference in slopes will not cross the non-inferiority margin of 0.15 UPDRS points per week.

### IV. Blinded Variance Estimate

To examine whether the variance estimates that were used in the sample size calculations are adequate, a blinded assessment of the variance magnitude will be performed after 1/3 of the subjects will complete the trial. A blinded evaluation of the early dropout rate will also be performed at this time point. If the variance estimates are larger by 10% or more than those projected, or the early dropout rate is 20% or higher, the sponsor may up-size the study via a protocol amendment.

### V. Secondary Efficacy Endpoint

Each statistically significant dose, as determined by the primary analysis, will be further tested, using the hierarchical method for multiple comparisons, comparing the early-start to the delayed-start rasagiline group, the change in Total UPDRS from baseline to LOV in the placebo controlled phase.

### Patient Selection

Untreated PD patients of either sex and any race could be included in this study. Diagnosis was based on having 2 cardinal signs (resting tremor, bradykinesia, rigidity), and if rest tremor was not present subjects must have unilateral onset with persistent asymmetry. Patients with atypical or secondary parkinsonism were excluded. Other entry criteria included disease duration of less than 18 months from time of diagnosis and a determination in the best judgment of the investigator that the patient would not require treatment in the subsequent 9 months. Patients with >3 weeks of treatment with any anti-parkinsonian medication prior to baseline were not eligible for the study. Prior use of rasagiline, selegiline, or coenzyme Q10 (in daily doses > 300 mg) within the previous 120 days was prohibited.

Since diagnostic error was a problem in the early PD population which might lead to the inclusion of subjects without idiopathic PD, a stringent set of inclusion and exclusion criteria was defined.

### Inclusion Criteria

Subjects must have met all the inclusion criteria to be eligible:
1. Men and women with idiopathic Parkinson's disease whose diagnosis is confirmed at screening, by the presence of at least two of the cardinal signs (resting tremor, bradykinesia, rigidity), without any other known or suspected cause of parkinsonism. If tremor is not present, subjects must have unilateral onset and persistent asymmetry.
2. Subjects with a diagnosis of early idiopathic PD of less than 1 years duration from time of documented diagnosis.
3. Subjects whose clinical condition at the time of study enrollment does not require any anti-PD treatment and, to the best of the investigator's judgment, will not require for the next 9 months.
4. Willing and able to give informed consent.

### Exclusion Criteria

Any of the following would exclude the subject from the study:
1. Subjects younger than 30 or older than 80 years of age.
2. Subjects with a loss of postural reflexes.
3. Subjects with a UPDRS Tremor score of 3 or greater in any limb.
4. Subjects with a Hoehn & Yahr Stage III or greater at screening.
5. Subjects with freezing while walking.
6. Subjects with any one of the following features that tended to exclude PD as the cause of Parkinsonism:
   a) History of repeated strokes with stepwise progression of Parkinsonian features
   b) History of repeated head injury or history of definite encephalitis
   c) Sustained remission
   d) Supranuclear gaze palsy
   e) Cerebellar signs
   f) Early severe autonomic involvement
   g) Babinski's sign
   h) Presence of a cerebral tumour or communicating hydrocephalus
   i) MPTP exposure
   j) Oculogyric crises
7. Subjects who had any previous use of rasagiline or selegiline.
8. Subjects who had any previous use of rasagiline or selegiline.
9. Subjects who used any other anti-PD medication (including anticholinergics) on a chronic (for more than 3 weeks) basis at any time prior to baseline.
10. Subjects who used any other anti-PD medication (including anticholinergics) for less than 3 weeks during the 3 month period prior to baseline, (not
   including a single L-Dopa dose as part of L-Dopa test).
11. Subjects who used any other anti-PD medication (including anticholinergics) for less than 3 weeks prior to the 3 month period preceding baseline whose anti-PD medication was intentionally ceased in order for the subject to enter the study.
12. Subjects who, based on the investigator's judgment, had clinically significant or unstable medical or surgical condition that may preclude safe and complete study participation. Such conditions included cardiovascular, vascular disease, pulmonary, hepatic impairment (Child-Pugh Score >5), renal, or metabolic diseases or malignancies as determined by medical history, physical examination, laboratory tests, chest x-ray, or ECG.
12. Hypertensive subjects whose BP, in the investigator's opinion, was not well controlled according to the subject's medical record or as observed during the week of home BP recording prior to baseline.
13. Subjects diagnosed with melanoma based on the screening dermatologic examination, or with a history of melanoma. Subjects with suspicious lesions at baseline who did not undergo biopsy.
14. Subjects with significant cognitive impairment as defined by MMSE score < 26.
15. Subjects with clinically significant psychiatric illness, including major depression [Beck Depression Inventory (short form) > 15].
16. Subjects with a history of alcohol or substance abuse within the past 2 years.
17. Subjects who took any experimental medications within 60 days prior to baseline.
18. Subjects who used coenzyme Q10 (in daily doses > 300 mg) within 120 days prior to baseline.
19. Subjects who used sympathomimetics (including over-the-counter remedies - nasal or oral), dextromethorphan, pethidine or St. John's Wort within the 7 days prior to baseline.
20. Subjects who used antidepressants, including selective serotonin reuptake inhibitors, tricyclic and tetracyclic antidepressants (except: amitriptyline < 50 mg/daily, or trazodone < 100 mg/daily, or citalopram < 20 mg/daily, or sertraline < 100 mg/daily or paroxetine < 30 mg/daily, or escitalopram < 10 mg/daily used as single drug therapy) within 42 days prior to baseline.
21. Subjects who used ciprofloxacin, a potent CYP 1A2 inhibitor within 7 days prior to baseline.
22. Subjects who used MAO inhibitors including reserpine or methyldopa within the three months prior to baseline, or treatment with an anti-emetic or antipsychotic medication with central dopamine antagonist activity within the six months prior to baseline.
23. Women who were not postmenopausal, surgically sterilized, or using adequate birth control [oral birth control pills, 1UD, or a long acting injectable form of contraception; barrier methods alone (i.e., condom) are not sufficient]. Women of childbearing potential without a negative pregnancy test (serum beta-HCG) at screening.

A total of 1176 patients were randomized to a treatment group. Baseline demographics were provided in table 1. The mean age of subjects was 62.2 ± 9.6 years, there were 718 males (61.1%) and 458 women (38.9%), mean time from diagnosis was 4.5 ± 4.6 months, and the mean total UPDRS score was 20.4 ± 8.5. There were 85 terminations during the placebo phase (7%).

**Table 1: Baseline Demographics (1176 patients)**

| | **Mean** | **SD** | **Min** | **Median** | **Max** |
|---|---|---|---|---|---|
| Age (years) - All | 62.2 | 9.6 | 31.2 | 63.0 | 80.9 |
| Time from PD diagnosis (months) | 4.5 | 4.6 | 0.0 | 2.7 | 18.0 |
| UPDRS Total (range: 0 - 176) | 20.4 | 8.5 | 3.0 | 19.0 | 53.0 |
| UPDRS Part I (range: 0 - 16) | 1.0 | 1.2 | 0.0 | 1.0 | 8.0 |
| UPDRS Part II (range: 0 - 52) | 5.2 | 3.0 | 0.0 | 5.0 | 17.0 |
| UPDRS Part III (range: 0 - 108) | 14.2 | 6.4 | 2.0 | 13.5 | 39.5 |
| Modified Hoehn and Yahr | 1.5 | 0.5 | 1.0 | 1.5 | 2.5 |

### Study Assessment

Visits are performed at time points indicated in Figure 1. At each visit except at week 4, a UPDRS evaluation is performed. Other evaluations performed at each visit included measures of quality of life, adverse events reporting, and standard laboratory assessments. Subjects were assessed on the Total UPDRS by the same investigator at all study visits.

### Safety and Tolerability

Tolerability is assessed by the number of subjects (%) who discontinue the study and the number of subjects who discontinue the study due to adverse events (AEs). Safety assessments include incidence of adverse events, laboratory values, vital signs, home blood pressure monitoring, ECG, physical and neurological examination and skin examination by a qualified dermatologist.

### Results and Discussions

### 1. Comparison of Slopes in Weeks 12-36 of Placebo Controlled (PC) Phase

a) Randomization was performed for 4 study arms
b) Groups were comparable at baseline
c) Analyses were based on one placebo arm according to the SAP
d) Slopes separation analyses:
   i) Predefined model assumed linearity across weeks 12, 24 and 36
   ii) Linearity criteria was not met
   iii) Results were confirmed using alternative
      categorical model

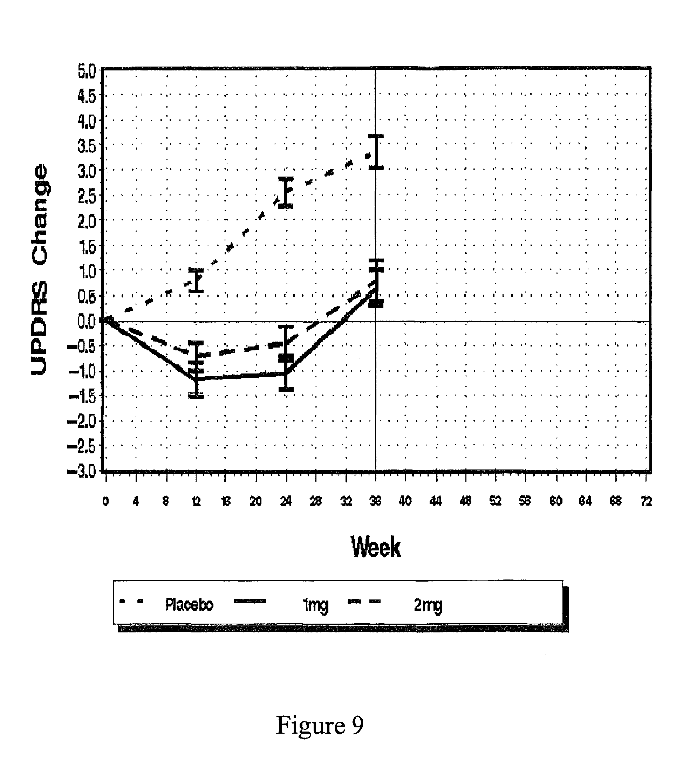

Figure 9 shows the changes in Total UPDRS scores from baseline in each of visit weeks 12, 24 and 36 during Phase I of the study.

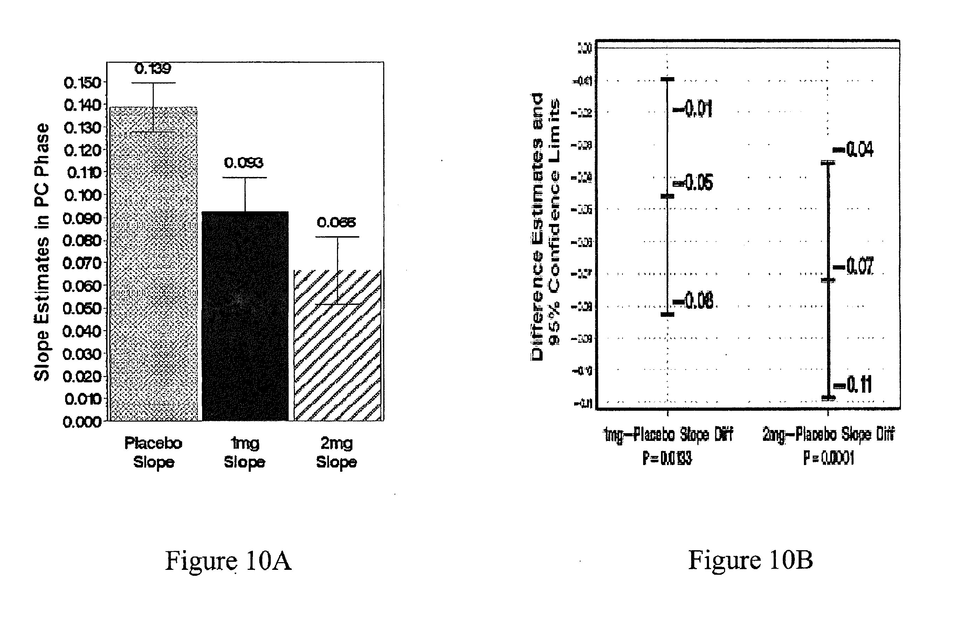

Figure 10A and 10B show comparison of the slopes during Weeks 12-36 of Phase I of the study.

Table 2 below compares the slopes in the PC phase (weeks 12 - 36) for placebo, 1mg, and 2mg groups.

**Table 2 - Comparison of Slopes in the PC phase (weeks 12 - 36) - Results for ITT CO and PP Data Analyses Sets**

| **Model that assumes linearity** | | | | | | | |
|---|---|---|---|---|---|---|---|
| **Data Analysis Set** | **No. of Subjects** | **Comparison** | **Estimate** | **SE** | **P-Value** | **Lower 95% CI Limit** | **Upper 95% CI Limit** |
| **ITT** | **1174** | **1mg-Placebo Slope Difference** | **-0.046** | **0.019** | **0.0133** | **-0.083** | **-0.010** |
| | | **2mg-Placebo Slope Difference** | **-0.072** | **0.019** | **0.0001** | **-0.109** | **-0.036** |
| **CO** | **840** | **1mg-Placebo Slope Difference** | **-0.033** | **0.018** | **0.0645** | **-0.069** | **0.002** |
| | | **2mg-Placebo Slope Difference** | **-0.061** | **0.018** | **0.0007** | **-0.096** | **-0.026** |
| **PP** | **834** | **1mg-Placebo Slope Difference** | **-0.037** | **0.018** | **0.0414** | **-0.072** | **-0.001** |
| | | **2mg-Placebo Slope Difference** | **-0.065** | **0.018** | **0.0003** | **-0.100** | **-0.030** |

| **Alternative Categorical Model that Compares week 36 to week 12** | | | | | | | |
|---|---|---|---|---|---|---|---|
| **Data Analysis Set** | **No. of Subjects** | **Comparison** | **Estimate** | **SE** | **P-Value** | **Lower 95% CI Limit** | **Upper 95% CI Limit** |
| **ITT** | **1174** | **1mg-Placebo Week 36 - 12** | **-1.043** | **0.446** | **0.0196** | **-1.918** | **-0.168** |
| | | **2mg-Placebo Week 36 - 12** | **-1.668** | **0.445** | **0.0002** | **-2.541** | **-0.794** |
| **CO** | **840** | **1mg-Placebo Week 36 - 12** | **-0.799** | **0.434** | **0.0659** | **-1.650** | **0.053** |
| | | **2mg-Placebo Week 36 - 12** | **-1.463** | **0.430** | **0.0007** | **-2.307** | **-0.620** |
| **PP** | **834** | **1mg-Placebo Week 36 - 12** | **-0.873** | **0.465** | **0.0605** | **-1.785** | **0.038** |
| | | **2mg-Placebo Week 36 - 12** | **-1.553** | **0.461** | **0.0008** | **-2.458** | **-0.648** |

### 2. Change from Baseline to Last Observed Values (LOV) in Total UPDRS scores at Week 36 of PC Phase

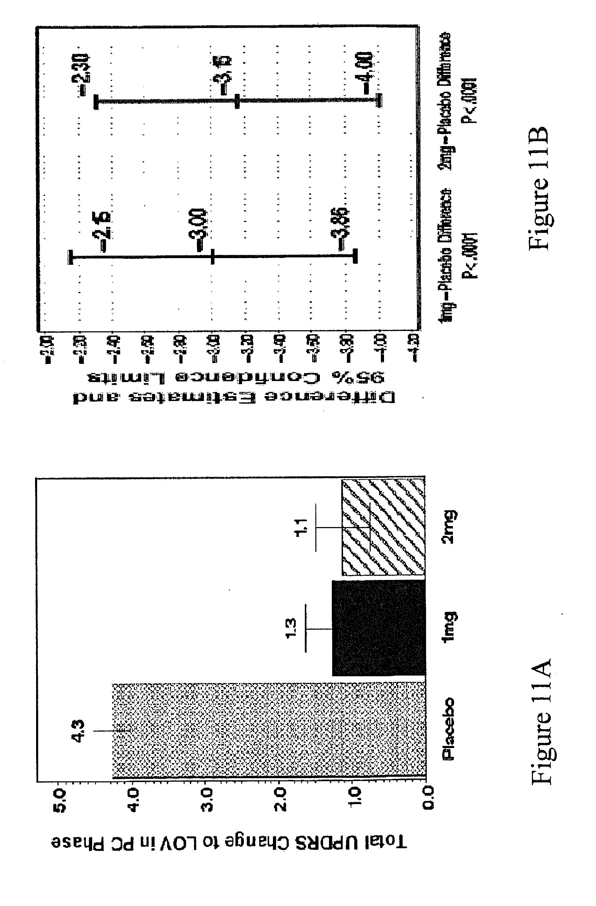

Figure 11A and 11B show the total UPDRS changes to LOV at Week 36 for placebo, 1mg, and 2mg groups.

### 3. Comparison at Week 72 (end of the Active Phase)

Comparison between Delayed and Early Start were performed separately for 1mg and 2mg since the 2 doses behaved differently

Figure 12 shows the change in total UPDRS score at Weeks 12, 24, 36, 42, 48, 54, 60, 66 and 72 for 1mg Delayed Start and 1mg Early Start groups.

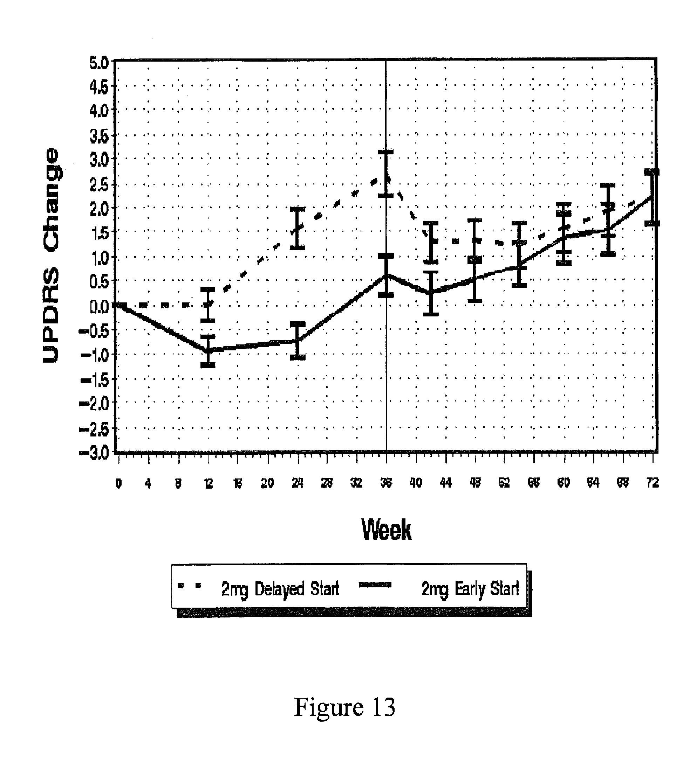

Figure 13 shows the change in total UPDRS score at Weeks 12, 24, 36, 42, 48, 54, 60, 66 and 72 for 2mg Delayed Start and 2mg Early Start groups.

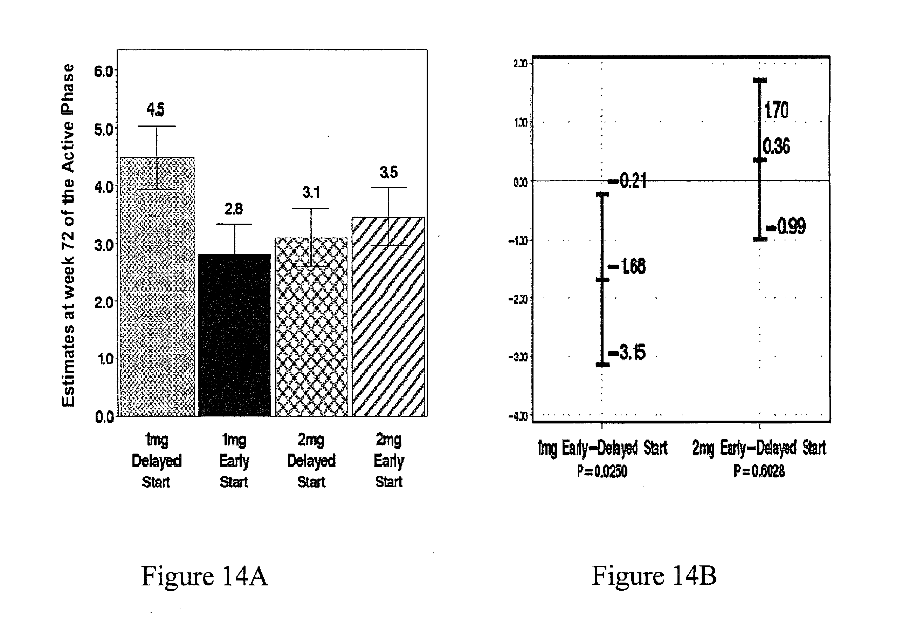

Figure 14A and 14B show Adjusted Means at Week 72 for 1mg Delayed Start, 1mg Early Start groups, 2mg Delayed Start, and 2mg Early Start groups.

Table 3 below shows comparison of at Week 72 for 1mg Delayed Start, 1mg Early Start groups, 2mg Delayed Start, and 2mg Early Start groups.

**Table 3: Comparison at Week 72 (end of the Active Phase)**

| **Data Analysis Set** | **No. of Subjects** | **Comparison** | **Estimate** | **SE** | **P-Value** | **Lower 95% CI Limit** | **Upper 95% CI Limit** |
|---|---|---|---|---|---|---|---|
| **ACTE** | **489** | **1mg Early-Delayed Start at week 72** | **-1.680** | **0.747** | **0.0250** | **-3.148** | **-0.212** |
| | **507** | **2mg Early-Delayed Start at week 72** | **0.356** | **0.684** | **0.6028** | **-0.989** | **1.702** |
| **CO** | **409** | **1mg Early-Delayed Start at week 72** | **-1.592** | **0.762** | **0.0374** | **-3.090** | **-0.093** |
| | **431** | **2mg Early-Delayed Start at week 72** | **-0.088** | **0.681** | **0.8974** | **-1.426** | **1.250** |
| **PP** | **405** | **1mg Early-Delayed Start at week 72** | **-1.594** | **0.770** | **0.0392** | **-3.108** | **-0.079** |
| | **429** | **2mg Early-Delayed Start at week 72** | **-0.024** | **0.679** | **0.9723** | **-1.359** | **1.312** |

### 4. Non-inferiority of Slopes in the Active Phase (Weeks 48-72)

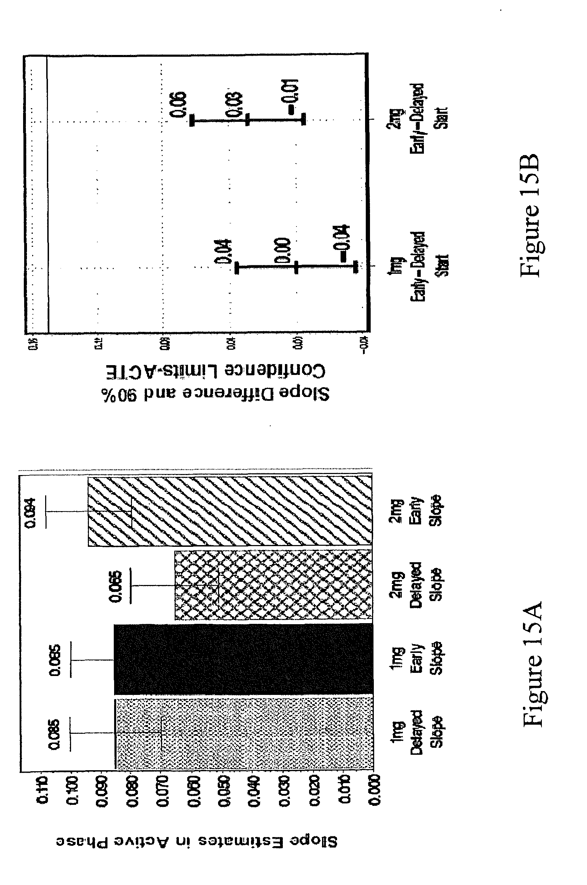

Figure 15A and 15B show slope estimates during Weeks 48-72 for 1mg Delayed Start, 1mg Early Start groups, 2mg Delayed Start, and 2mg Early Start groups.

Table 4 below shows non-Inferiority of slopes in the active phase during Weeks 48-72 for 1mg Delayed Start, 1mg Early Start groups, 2mg Delayed Start, and 2mg Early Start groups.

**Table 4 - Non-Inferiority of Slopes (0.15 margin) in the Active Phase Week 48-72 - Results for ITT CO and PP Data Analyses Sets**

| | | | **Estimate** | **Lower 95% CI Limit** | **Upper 95% CI Limit** |
|---|---|---|---|---|---|
| **Data Analysis Set** | **No. of Subjects** | **Comparison** | | | |
| **ACTE** | **489** | **1mg Early-Delayed Start Slope Difference** | **0.000** | **-0.036** | **0.036** |
| | **507** | **2mg Early-Delayed Start Slope Difference** | **0.029** | **-0.005** | **0.062** |
| **CO** | **409** | **1mg Early-Delayed Start Slope Difference** | **-0.011** | **-0.047** | **0.025** |
| | **431** | **2mg Early-Delayed Start Slope Difference** | **0.021** | **-0.010** | **0.053** |
| **PP** | **405** | **1mg Early-Delayed Start Slope Difference** | **-0.011** | **-0.047** | **0.024** |
| | **429** | **2mg Early-Delayed Start Slope Difference** | **0.022** | **-0.010** | **0.053** |

### 5. Additional Efficacy Endpoints Analyses

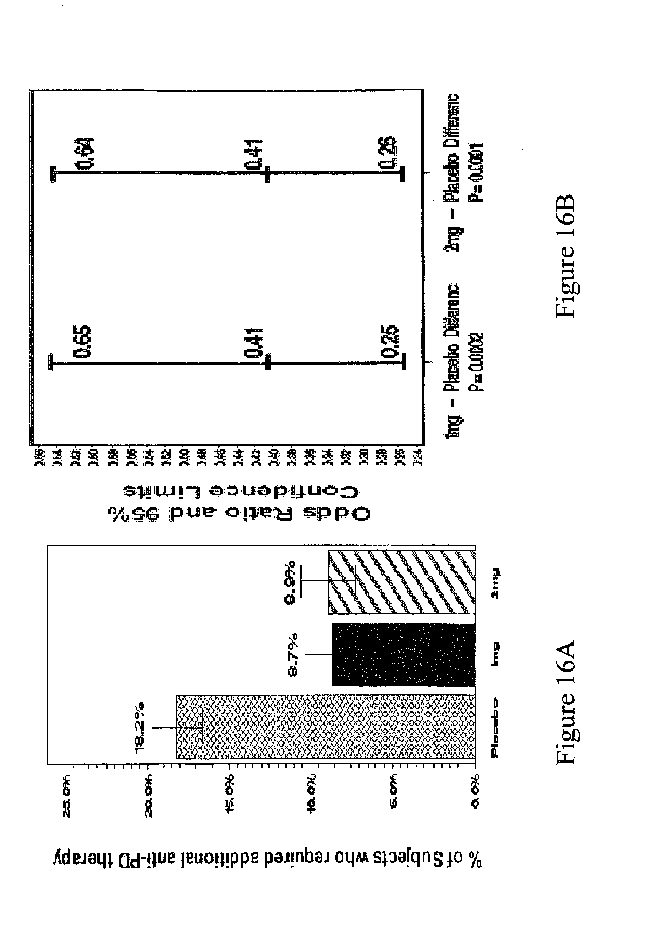

Figure 16A and 16B show % of subjects who required additional anti-PD therapy during PD Phase for placebo, 1mg and 2mg groups.

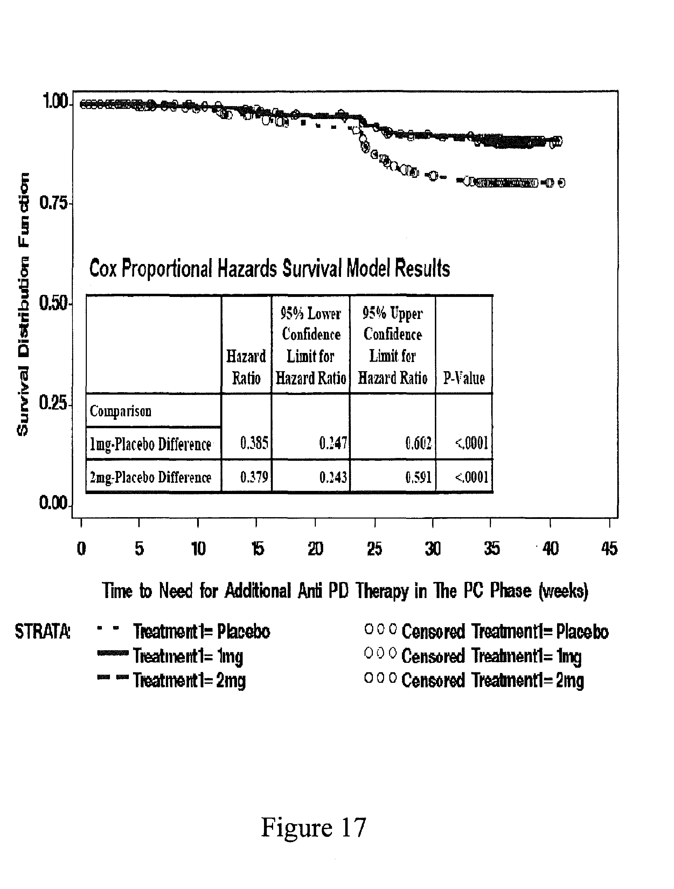

Figure 17 shows time to additional anti-PD therapy during PD Phase for placebo, 1mg and 2mg groups.

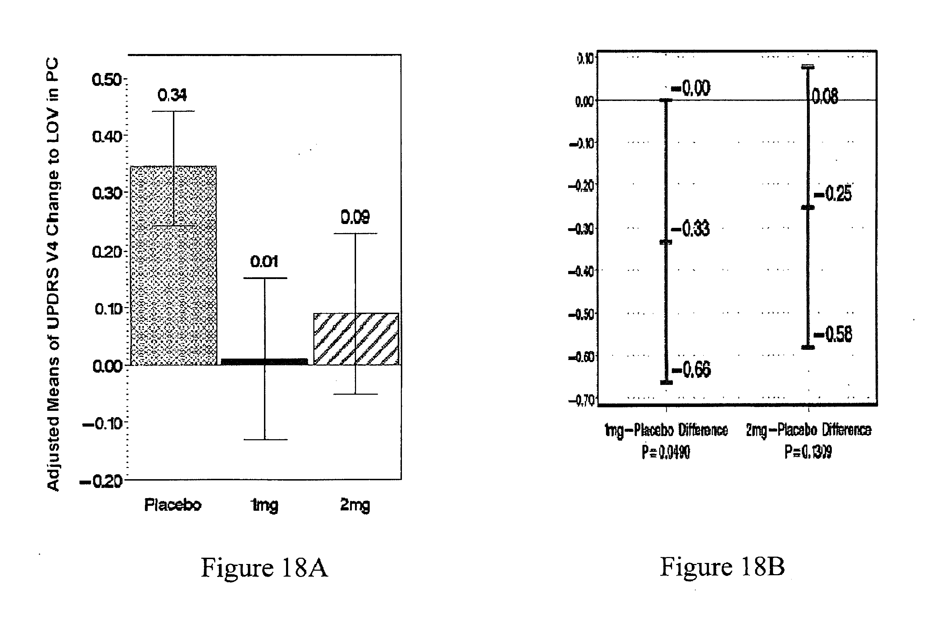

Figure 18A and 18B show adjusted means of the change from baseline to Last Observed Value ("LOV") in Part I of UPDRS version 4 (Non-Motor Aspects of Experiences of Daily Living) in PC Phase for placebo, 1mg and 2mg groups.

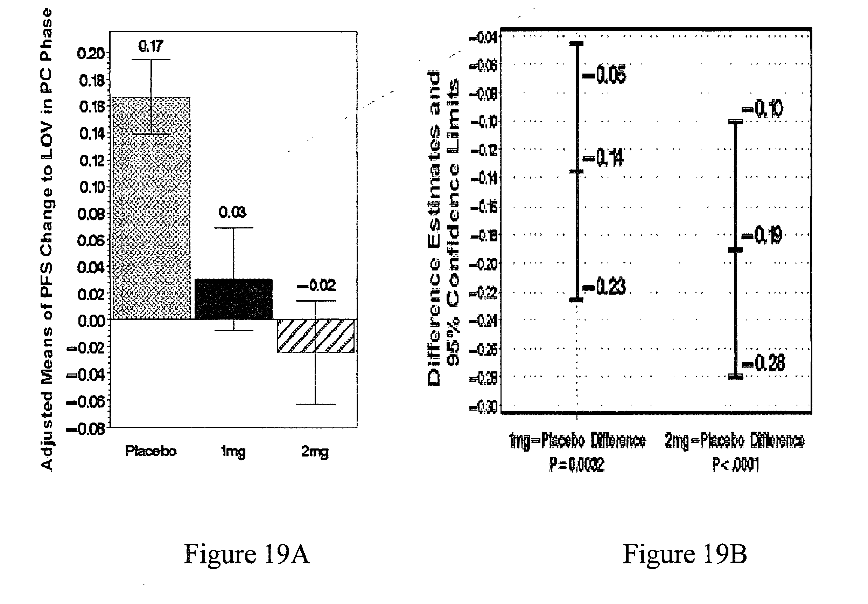

Figure 19A and 19B show adjusted means of the change from baseline to LOV in Parkinson Fatigue Scale (PFS) in PC Phase for placebo, 1mg and 2mg groups.

Table 5 below compares time to additional anti-PD therapy in the PC Phase for placebo, 1mg and 2mg groups.

**Table 5. Time to additional anti-PD therapy in the PC Phase - Kaplan Meier Curves and Cox Proportional Hazards Model Results**

| | **Hazard Ratio** | **95% Lower Confidence Limit for Hazard Ratio** | **95% Upper Confidence Limit for Hazard Ratio** | **P-Value** |
|---|---|---|---|---|
| **Comparison** | **0.385** | **0.247** | **0.602** | **<.0001** |
| **1mg-Placebo Difference** | | | | |
| **2mg-Placebo Difference** | **0.379** | **0.243** | **0.591** | **<.0001** |

The above results show that:
a) The symptomatic effect observed in TEMPO was replicated in ADAGIO placebo controlled (PC) phase for both rasagiline 1mg and 2mg versus placebo;
b) Disease modifying effect was demonstrated according to FDA requirements as predefined in the statistical analysis plan for the 1mg treatment;
c) Disease modifying effect was not evident for the 2mg treatment;
d) The beneficial effect of rasagiline 1mg and 2mg was also demonstrated at the end of the PC phase for:
   i) Need for additional anti PD Therapy
   ii) Parkinson Fatigue Scale
   iii)UPDRS Version 4 Part 1 (1mg only)

The results described herein also show that treatment with rasagiline in an early stage PD patient presents a slower rate of progression, when compared to other early stage PD patients. Positive results in current study demonstrated that early treatment with rasagiline provides benefits that cannot be attained with later initiation of the drug, and are sustained for at least 18 months.

The results described herein further show that administration of rasagiline to Early Stage PD patients delays the rate of progression of PD. The rate of progression has improved from 0.139 score (change in Total UPDRS)/week to 0.066 score (change in Total UPDRS)/week.

The results herein also show that administration of rasagiline to Early Stage PD patients maintain the initial difference in change in Total UPDRS score when compared to early stage PD patients receiving delayed treatment. The difference in change in Total UPDRS score is maintained at 1.7 score between Week 48 and Week 72.

The results herein also show that administration of rasagiline to Early Stage PD patients reduces the change in Total UPDRS score at week 72. The change in Total UPDRS score at week 72 was 2.8 corresponding to a reduction of 62.2% in Total UPDRS score.

Therefore, the results herein show that administration of rasagiline to Early Stage PD patients demonstrates disease modifying effect.

### 6. Analysis of Results of 2mg Dose

Rasagiline 1 mg/day early-start met all endpoints of the primary end point analysis: less deterioration in UPDRS points/week than placebo between weeks 12 and 36 (early-start=0.09±0.02, placebo=0.14±0.01; P=0.013); less worsening than delayed-start in UPDRS score between baseline and week 72 (early-start=2.82±0.53, delayed-start=4.52±0.56; P=0.025), and non-inferiority to delayed-start in deterioration in UPDRS ponts/week between weeks 48 and 72 (early-start=0.085±0.02, delayed-start=0.085±0.02; P<0.001). Rasagiline 2 mg/day early-start did not meet all endpoints as worsening in UPDRS score between baseline and week 72 was not less than delayed-start (early-start=3.47±0.5, delayed-start=3.11±0.5; P=0.6).

The primary analysis was comprised of three hierarchal endpoints based on total UPDRS score. The first endpoint compared slope estimates (change in UPDRS units/week) between the rasagiline (1 or 2 mg/day) and placebo groups from weeks 12-36. This determined if there was a difference in the rate of progression of UPDRS score between each rasagiline group and placebo after week 12, when it was assumed that the full symptomatic effect of rasagiline had been established. A disease modifying agent would be expected to slow the rate of progression compared to placebo. The second endpoint compared estimated change in total UPDRS score between baseline and week 72 in the rasagiline (1 or 2 mg/day) early-start and delayed-start groups. This determined if benefits observed in the early-start group at the end of phase I were still present at the end of the study when subjects in early- and delayed-start groups were receiving the same treatment. The third endpoint tested for non-inferiority of UPDRS slope estimates between weeks 48-72 in the early- and delayed-start groups. A non-inferiority margin of 0.15 UPDRS units/week was pre-specified. This endpoint was designed to determine if the difference between groups was enduring (as would be expected with a disease-modifying effect) and not diminishing (as would be expected with a symptomatic agent). For each dose, all three endpoints had to be met to declare the study positive.

The secondary endpoint was change in total UPDRS score between baseline and last observed value in Phase I. Sample size was based on the TEMPO study, and indicated that 1100 subjects would provide 87% power to detect a difference ≥1.8 UPDRS points between early- and delayed-start groups in mean change from baseline to weeks 48-72 with alpha=0.05 and 15% dropout.

For the first primary endpoint, all patients with evaluations at baseline and week 12 or later were included in the analysis. For the second and third primary endpoints all subjects with at least 24 weeks of treatment during phase I and an evaluation at the week 48 visit or later were included. Safety assessments included all randomized patients.

Statistical analysis was performed with a mixed model repeated measures analysis of covariance that included the following fixed effects: treatment group, week in trial, week by treatment interaction, center, and baseline total UPDRS score. The first endpoint was analyzed using the combined placebo group. For endpoints two and three, the model was fitted separately for each dosage because heterogeneous covariate effects were observed between the two dosages. To maintain an experimentwise type I error of .05, the hierarchal method was used to account for multiple primary endpoints within each dose and the Hochberg step-up Bonferroni method was used to account for testing two doses. This allowed for each dose to be tested separately. Various sensitivity and supportive analyses including multiple imputation strategies were employed to validate the result and address missing data. For the secondary endpoint, an ANCOVA was used to assess the adjusted mean change in total UPDRS score between baseline and last observed value in Phase I.

To address the possibility that a symptomatic effect might mask a disease-modifying effect in this very mild cohort of patients, a post-hoc subgroup analysis was conducted in subjects with high (upper-quartile) baseline total UPDRS scores.
1164 (99%) subjects were included in the first primary endpoint analysis, and 996 (85%) were included in the second and third primary endpoint analyses. There were no significant demographic differences between treatment groups. Mean disease duration from time of diagnosis was 4.5 months and mean total UPDRS was 20.4. Results of the three endpoints comprising the primary analysis and the secondary endpoint for each dose are provided in Table 6 below.

**Table 6. ADAGIO results (±SE) for the primary and the secondary endpoints**

| | | **Result** | **S.E.** | **P-value** | **95% C.I.** |
|---|---|---|---|---|---|
| **Primary 1 (rate of change in UPDRS units/wk, weeks 12-36)** | | | | | |
| | Placebo | 0.14 | 0.01 | | |
| | 1 mg/day | 0.09 | 0.02 | | |
| | 2 mg/day | 0.07 | 0.02 | | |
| | 1 mg/day - placebo | -0.05 | 0.02 | 0.01 | -0.08,-0.01 |
| | 2 mg/day - placebo | -0.07 | 0.019 | <0.001 | -0.11,-0.04 |

| **Primary 2 (estimated change in total UPDRS from baseline to week 72)** | | | | | |
|---|---|---|---|---|---|
| | 1 mg/day early-start | 2.82 | 0.53 | | |
| | 1 mg/day delayed-start | 4.50 | 0.56 | | |
| | 2 mg/day early-start | 3.47 | 0.50 | | |
| | 2 mg/day delayed-start | 3.11 | 0.50 | | |
| | 1 mg/day early-start - delayed-start | -1.68 | 0.75 | 0.025 | -3.15,-0.21 |
| | 2 mg/day early-start - delayed-start | 0.36 | 0.68 | 0.60 | -0.99, 1.70 |

| **Primary 3 (rate of change in UPDRS units/wk, weeks 48-72)** | | | | | |
|---|---|---|---|---|---|
| | 1 mg/day early-start | 0.085 | 0.02 | | |
| | 1 mg/day delayed-start | 0.085 | 0.02 | | |
| | 2 mg/day early-start | 0.094 | 0.01 | | |
| | 2 mg/day delayed-start | 0.065 | 0.02 | | |
| | 1 mg/day early-start - delayed-start | 0.00 | 0.02 | <0.001 | -0.04,0.04* |
| | 2 mg/day early-start - delayed start | 0.03 | 0.02 | <0.001 | -0.01,0.06* |

| **Secondary Endpoint (change in total UPDRS from baseline to final visit in phase I)** | | | | | |
|---|---|---|---|---|---|
| | Placebo | 4.27 | 0.26 | | |
| | 1 mg/day | 1.26 | 0.36 | | |
| | 2 mg/day | 1.11 | 0.36 | | |
| | 1 mg/day - placebo | -3.01 | 0.43 | <0.001 | -3.86,-2.15 |
| | 2 mg/day - placebo | -3.15 | 0.43 | <0.001 | -4.00,-2.31 |

| | | | | | |
|---|---|---|---|---|---|
| 1164 subjects were included in first primary endpoint analysis; 996 subjects were included in the second and third primary endpoint analyses. *Non-inferiority of early-start group slope over delayed-start group is achieved if the upper limit of one-sided 95% CI (90% CI) for difference in slopes does not cross the margin of 0.15 UPDRS points per week | | | | | |

### Rasagiline 1 mg/day

As shown in Table 6, the week 12-36 slope estimates demonstrate a slower rate of UPDRS deterioration for rasagiline versus placebo (-0.05±0.02; P=0.01). The early-start group had less deterioration between baseline and week 72 in mean total UPDRS score than the delayed-start group (-1.68±0.75; P=0.025). There was non-inferiority of slope estimates between weeks 48-72 for the early- and delayed-start groups (0.00; P<0.001; 90%CI:[-0.04,0.04]). Thus, rasagiline 1 mg/day met all three primary analysis endpoints. The model for the first primary endpoint assumed linearity in change in UPDRS units/week; results were confirmed by an alternative categorical model. The results of the second primary endpoint were confirmed by several predefined sensitivity and confirmatory analyses.

As shown in Table 7 below, for the secondary endpoint (change in total UPDRS score between baseline and last observed value in phase I), rasagiline 1mg/day was superior to placebo (-3.01±0.43; P<0.001).

**Table 7: Confirmatory analyses for change from baseline to week 72 in Total-UPDRS scores for rasagiline 1 mg/day early- and delayed-start groups**

| Dataset | Analysis | Estimate | SE | p-value | 95% CI |
|---|---|---|---|---|---|
| **Primary study cohort (n=489)** | **MMRM (week 48-72)** | **-1.7** | **0.7** | **0.025** | **-3.1, -0.2** |
| Completers (n=409)* | MMRM (week 48-72) | -1.6 | 0.8 | 0.04 | -3.1, -0.1 |
| Per protocol (n=405) | MMRM (week 48-72) | -1.6 | 0.8 | 0.04 | -3.1, -0.1 |
| Imputed - primary study cohort* | Multiple imputation (week 48-72) | -1.8 | 0.7 | 0.01 | -3.2, -0.4 |
| Imputed - primary study cohort* | Imputation by means of the delayed-start group (week 48-72) | -1.2 | 0.6 | 0.04 | -2.3, -0.04 |
| Primary study cohort | ANCOVA with LOCF | -1.7 | 0.7 | 0.01 | -3.1, -0.4 |
| Primary study cohort | MMRM; average change from baseline across weeks 48-72 | -1.6 | 0.6 | 0.01 | -2.7, -0.5 |
| ITT cohort (observed data) | MMRM (weeks 12-72) | -1,7 | 0.8 | 0.03 | -3.2, -0.2 |

| | | | | | |
|---|---|---|---|---|---|
| The primary analysis is in bold. * indicates a predefined sensitivity analyses MMRM = Mixed Model Repeated Measures Model with categorical week in trial ANCOVA = Analysis of covariance LOCF = last-observation-carried-forward | | | | | |

### Rasagiline 2 mg/day

As also shown in Table 6, slope estimates between weeks 12 and 36 show less deterioration in UPDRS score in rasagiline compared to placebo groups (-0.07±0.02; P<0.001). However, deterioration in total UPDRS score between baseline and week 72 was not significantly different between the early- and delayed-start groups (0.36±0.68; P=0.60). There was non-inferiority of slope estimates between weeks 48-72 for the early- and delayed-start groups (0.03; P<0.001; 90% CI:[-0.01,0.06]). Thus, rasagiline 2 mg/day did not meet all three endpoints of the primary analysis and the results are considered to be negative for this dose. For the secondary endpoint, rasagiline was superior to placebo (-3.15±0.43; P<0.001).

### Post-hoc subgroup analysis

To address the possibility that a symptomatic effect of rasagiline 2mg/day might have masked a disease-modifying benefit in patients with very mild UPDRS scores, the primary and secondary analyses were performed on subjects in the upper quartile of baseline total UPDRS scores (>25.5). For patients receiving rasagiline 2 mg/day, the difference in UPDRS deterioration between baseline and week 72 in the early- and delayed-start groups was significantly different for subjects with baseline UPDRS scores in the upper quartile versus those in the other three quartiles (P=0.03). This interaction suggests that these subgroups can be considered separately.

**Table 8. ADAGIO Results (±SE) of Primary and the Secondary Analyses For patients with Baseline UPDRS > 25.5 (Upper Quartile)**

| | | **Result** | **S.E.** | **P-value** | **95% C.I.** |
|---|---|---|---|---|---|
| **Primary 1 (rate of change in UPDRS units/wk; weeks 12-36)** | | | | | |
| | Placebo | 0.25 | 0.03 | | |
| | 1 mg/day | 0.14 | 0.04 | | |
| | 2 mg/day | 0.05 | 0.04 | | |
| | 1 mg/day - placebo | -0.11 | 0.05 | 0.03 | -0.21,-0.01 |
| | 2 mg/day - placebo | -0.20 | 0.05 | <0.001 | -0.30,-0.11 |

| **Primary 2 (estimated change in total UPDRS from baseline to week 72)** | | | | | |
|---|---|---|---|---|---|
| | 1 mg/day early-start | 2.01 | 1.09 | | |
| | 1 mg/day delayed-start | 5.41 | 1.20 | | |
| | 2 mg/day early-start | 1.46 | 1.15 | | |
| | 2 mg/day delayed-start | 5.09 | 1.19 | | |
| | 1 mg/day early-start - delayed-start | -3.40 | 1.66 | 0.04 | -6.7,-0.1 |
| | 2 mg/day early-start - delayed-start | -3.63 | 1.72 | 0.04 | -7.0,-0.2 |

| **Primary 3 (rate of change in UPDRS units/wk; weeks 48-72)** | | | | | |
|---|---|---|---|---|---|
| | 1 mg/day early-start | 0.075 | 0.04 | | |
| | 1 mg/day delayed-start | 0.095 | 0.04 | | |
| | 2 mg/day early-start | 0.106 | 0.04 | | |
| | 2 mg/day delayed-start | 0.137 | 0.04 | | |
| | 1 mg/day early-start - delayed-start | -0.02 | 0.05 | <0,001 | -0.11,0.07* |
| | 2 mg/day early-start - delayed start | -0.03 | 0.06 | <0.001 | -0.13,0.06* |

| **Secondary Endpoint (change in total UPDRS from baseline to final visit in phase I)** | | | | | |
|---|---|---|---|---|---|
| | Placebo | 6.70 | 0.57 | | |
| | 1 mg/day | 0.27 | 0.82 | | |
| | 2 mg/day | -0.43 | 0.80 | | |
| | 1 mg/day - placebo | -6.43 | 0.97 | <0.001 | -8.3,-4.5 |
| | 2 mg/day - placebo | -7.13 | 0.96 | <0.001 | -9.0,-5.2 |

| | | | | | |
|---|---|---|---|---|---|
| 286 subjects were included in first primary endpoint analysis; 219 subjects were included in the second and third primary endpoint analyses. *Non-inferiority of early-start group slope over delayed-start group is achieved if the upper limit of one-sided 95% CI (90% CI) for difference in slopes does not cross the margin of 0.15 UPDRS points per week | | | | | |

**Table 9: ADAGIO Results (±SE) of Primary and the Secondary Analyses For patients with Baseline UPDRS ≤ 25.5 (Lower 3 Quartiles)**

| | | **Result** | **S.E.** | **P-value** | **95% C.I.** |
|---|---|---|---|---|---|
| Primary 1 (rate of change in UPDRS units/wk; weeks 12-36) | | | | | |
| | Placebo | 0.11 | 0.01 | | |
| | 1 mg/day | 0.08 | 0.02 | | |
| | 2 mg/day | 0.07 | 0.02 | | |
| | 1 mg/day - placebo | -0.03 | 0.02 | 0.13 | -0.07, 0.01 |
| | 2 mg/day - placebo | -0.03 | 0.02 | 0.07 | -0.07, 0.003 |

| Primary 2 (estimated change in total UPDRS from baseline to week 72) | | | | | |
|---|---|---|---|---|---|
| | 1 mg/day early-start | 2.94 | 0.47 | | |
| | 1 mg/day delayed-start | 4.03 | 0.51 | | |
| | 2 mg/day early-start | 3.70 | 0.49 | | |
| | 2 mg/day delayed-start | 2.60 | 0.51 | | |
| | 1 mg/day early-start- delayed-start | -1.08 | 0.66 | 0.10 | -2.39, 0.22 |
| | 2 mg/day early-start - delayed-start | 1.10 | 0.68 | 0.11 | -0.24, 2.44 |

| Primary 3 (rate if change in UPDRS units/wk; weeks 48-72) | | | | | |
|---|---|---|---|---|---|
| | 1 mg/day early-start | 0.09 | 0.02 | | |
| | 1 mg/day delayed-start | 0.08 | 0.02 | | |
| | 2 mg/day early-start | 0.09 | 0.02 | | |
| | 2 mg/day delayed-start | 0.05 | 0.02 | | |
| | 1 mg/day early-start - delayed-start | 0.004 | 0.02 | <0.001 | -0.04, 0.04* |
| | 2 mg/day early-start - delayed start | 0.05 | 0.02 | <0.001 | 0.01, 0.08* |

| Secondary Endpoint (change in total UPDRS from baseline to final visit in phase I) | | | | | |
|---|---|---|---|---|---|
| | Placebo | 3.74 | 0.28 | | |
| | 1 mg/day | 1.40 | 0.38 | | |
| | 2 mg/day | 1.58 | 0.39 | | |
| | 1 mg/day - placebo | -2.34 | 0.45 | <.001 | -3.21, -1.4 |
| | 2 mg/day - placebo | -2.16 | 0.46 | <.001 | -3.06,-1.26 |

| | | | | | |
|---|---|---|---|---|---|
| 878 subjects were included in first primary endpoint analysis; 777 subjects were included in the second and third primary endpoint analyses. *Non-inferiority of early-start group slope over delayed-start group is achieved if the upper limit of one-sided 95% CI (90% CI) for difference in slopes does not cross the margin of 0.15 UPDRS points per week | | | | | |

Subjects in the upper quartile of both doses met all 3 primary endpoints, as shown in Table 8. For the rasagiline 2mg/day upper-quartile subgroup, early-start subjects had less deterioration in UPDRS score between baseline and week 72 than delayed-start subjects (-3.63±1.72; P=0.038; N=114). Early-start subjects in the upper quartile treated with rasagiline 1mg/day, also had less deterioration in total UPDRS between baseline and week 72 than delayed-start subjects (-3.40±1.66; P=0.044, N=105). For the subgroup of patients in the lower three quartiles of baseline UPDRS scores (≤ 25.5), neither dose met all of the primary endpoints, as shown in Table 9.

It is possible that a more robust symptomatic effect of the 2 mg dose might have masked a benefit associated with early-start treatment in this very mild population of patients. Indeed, a post hoc subgroup analysis evaluating subjects in the quartile with the highest baseline UPDRS scores showed that early-start rasagiline 2mg/day provided significant benefits at 72 weeks in comparison to delayed-start (-3.63 UPDRS units) and all primary endpoints were met despite the relatively small sample size. This effect is illustrated in the following diagram:

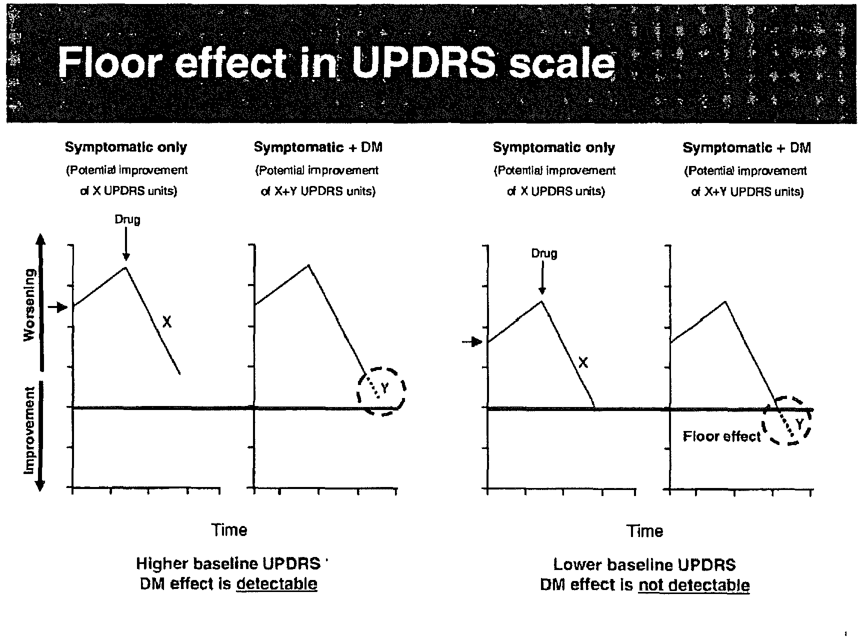

Further aspects and embodiments of the invention are set out in the following numbered clauses:
1. A method of reducing the rate of progression of Parkinson's disease in an early stage Parkinson's disease patient, the method comprising periodically administering to an early stage Parkinson's disease patient an amount of rasagiline, or a pharmaceutically acceptable salt of rasagiline effective to reduce the rate of progression of Parkinson's disease of the early stage Parkinson's disease patient.
2. The method of clause 1, wherein the patient is not receiving bromocriptine, benztropine, levodopa, ropinirole, pramipexole, rotigotine, cabergoline, entacapone, tolcapone, amantadine or selegiline.
3. The method of clause 1, wherein the patient is not receiving any therapy for Parkinson's disease other than rasagiline or a pharmaceutically acceptable salt of rasagiline.
4. The method of any one of clauses 1-3, wherein the rate of progression of Parkinson's disease is quantified by the Total UPDRS score, wherein an increase in the Total UPDRS score represents progression of Parkinson's disease symptoms, and the increment of the increase in Total UPDRS score over a period of time represents the rate of progression of Parkinson's disease.
5. The method of clause 4, wherein the period of time is 12, 24, or 36 weeks after initiation of administration of rasagiline or a pharmaceutically acceptable salt of rasagiline.
6. The method of any of clauses 1-5, wherein the rate of progression is an average Total UPDRS score increase of less than 0.15 units per week after the initial symptomatic effect period of the administration of rasagiline or a pharmaceutically acceptable salt of rasagiline.
7. The method of clause 6, wherein the rate of progression is an average Total UPDRS score increase of between 0.15 and 0.05 units per week after the initial symptomatic effect period of the administration of rasagiline or a pharmaceutically acceptable salt of rasagiline.
8. The method of clause 6, wherein the rate of progression is an average Total UPDRS score increase of between 0.15 and 0.07 units per week after the initial symptomatic effect period of the administration of rasagiline or a pharmaceutically acceptable salt of rasagiline.
9. The method of clause 6, wherein the rate of progression is an average Total UPDRS score increase of between 0.11 and 0.07 units per week after the initial symptomatic effect period of the administration of rasagiline or a pharmaceutically acceptable salt of rasagiline.
10. The method of clause 4, wherein the period of time is 48, 54, 60, 66 or 72 weeks after initiation of administration of rasagiline or a pharmaceutically acceptable salt of rasagiline.
11. The method of any one of clauses 1-10, wherein the amount of rasagiline administered is 1 mg per day.
12. The method of any one of clauses 1-11, wherein the pharmaceutically acceptable salt of rasagiline is rasagiline mesylate.
13. The method of any one of clauses 1-12, wherein the early stage Parkinson's disease patient is a Stage I patient according to Hoehn and Yahr rating.
14. The method of any one of clauses 1-12, wherein the early stage Parkinson's disease patient is a patient whose symptoms result in a UPDRS total score of less than 25.
15. The method of any one of clauses 1-12, wherein the early stage Parkinson's disease patient is a patient whose symptoms result in a UPDRS motor score of less than 17.5.
16. The method of any one of clauses 1-12, wherein the early stage Parkinson's disease patient is a patient whose symptoms have been diagnosed to indicate Parkinson's disease within the prior 12 months.
17. The method of any one of clauses 1-12, wherein the early stage Parkinson's disease patient is a patient whose symptoms have been diagnosed to indicate Parkinson's disease within the prior 6 months.
18. The method of any one of clauses 1-12, wherein the early stage Parkinson's disease patient is a patient whose symptoms have been diagnosed to indicate Parkinson's disease within the prior 1 month.
19. A method of reducing the rate of progression of Parkinson's disease in a Parkinson's disease patient, the method comprising periodically administering to the Parkinson's disease patient for more than 52 weeks an amount of rasagiline, or a pharmaceutically acceptable salt of rasagiline effective to reduce the rate of progression of Parkinson's disease.
20. The method of clause 19, wherein the patient is an early stage Parkinson's disease patient.
21. A method for delaying the need for symptomatic anti-Parkinsonian therapy in an early stage Parkinson's disease patient, comprising periodically administering to an early stage Parkinson's disease patient an amount of rasagiline or a pharmaceutically acceptable salt of rasagiline effective to delay the need for symptomatic anti-Parkinsonian therapy.
22. The method of clause 21, wherein the delay in the need for symptomatic anti-Parkinsonian therapy is more than 34 weeks after initiation of administration of rasagiline or a pharmaceutically acceptable salt of rasagiline.
23. The method of clause 21 or 22, wherein the amount of rasagiline administered is 1 mg per day.
24. The method of any one of clauses 21-23, wherein the pharmaceutically acceptable salt of rasagiline is rasagiline mesylate.
25. A method for reducing the risk of a Parkinson's disease patient requiring symptomatic anti-Parkinsonian therapy, comprising periodically administering to the patient for 36 weeks an amount of rasagiline or a pharmaceutically acceptable salt of rasagiline effective to reduce the risk of requiring symptomatic anti-Parkinsonian therapy.
26. The method of clause 25, wherein the administration is for more than 52 weeks.
27. The method of clause 25 or 26, wherein the risk is reduced by 40-60%.
28. The method of any one of clauses 25-27, wherein the amount of rasagiline or a pharmaceutically acceptable salt of rasagiline administered is 1 mg per day.
29. The method of any one of clauses 25-28, wherein the pharmaceutically acceptable salt of rasagiline is rasagiline mesylate.
30. A method of reducing the functional decline of an early stage Parkinson's disease patient, comprising identifying a patient to be an early stage Parkinson's disease patient, and periodically administering to the patient so identified an amount of rasagiline or a pharmaceutically acceptable salt of rasagiline effective to reduce the functional decline.
31. The method of clause 30, wherein functional decline of an early stage Parkinson's disease patient is quantified by the Total UPDRS score, an increase in the Total UPDRS score represents functional decline.
32. The method of clause 31, wherein the increase in Total UPDRS score is less than 3.97 units at 72 weeks after initiation of administration of rasagiline or a pharmaceutically acceptable salt of rasagiline.
33. The method of clause 30-32, wherein the amount of rasagiline administered is 1 mg per day.
34. The method of any one of clauses 30-33, wherein the pharmaceutically acceptable salt of rasagiline is rasagiline mesylate.
35. A method of reducing the functional decline in a Parkinson's disease patient, comprising periodically administering to the patient for more than 52 weeks an amount of rasagiline or a pharmaceutically acceptable salt of rasagiline effective to reduce the functional decline.
36. The method of clause 35, wherein functional decline is quantified by the Total UPDRS score, an increase in the Total UPDRS score represents functional decline.
37. The method of clause 36, wherein the increase in Total UPDRS score is less than 3.97 at 72 weeks after initiation of administration of rasagiline or a pharmaceutically acceptable salt of rasagiline.
38. The method of any one of clauses 35-37, wherein the amount of rasagiline administered is 1 mg per day.
39. The method of any one of clauses 35-38, wherein the pharmaceutically acceptable salt of rasagiline is rasagiline mesylate.
40. The method of any one of clauses 35-39, wherein the patient is an early stage Parkinson's disease patient.
41. A method of treating a patient exhibiting early signs of Parkinson's disease, comprising identifying a patient exhibiting early signs of Parkinson's disease, and periodically administering to the patient so identified an amount of rasagiline or a pharmaceutically acceptable salt of rasagiline effective to treat the patient.
42. The method of clause 41, wherein the amount of rasagiline or a pharmaceutically acceptable salt of rasagiline administered is 1 mg per day.
43. The method of clause 41 or 42, wherein the pharmaceutically acceptable salt of rasagiline is rasagiline mesylate.
44. A method of reducing fatigue in an early stage Parkinson's disease patient, comprising periodically administering to an early stage Parkinson's disease patient an amount of rasagiline or a pharmaceutically acceptable salt of rasagiline effective to reduce fatigue.
45. The method of clause 44 wherein the Parkinson's Fatigue Scale is reduced by between 0.05 and 0.23 compared to a patient who is not being treated by rasagiline.
46. A method of reducing severity of non-motor symptoms in an early stage Parkinson's disease patient, comprising periodically administering to an early stage Parkinson's disease patient an amount of rasagiline or a pharmaceutically acceptable salt of rasagiline effective to reduce the severity of non-motor symptoms.
47. The method of clause 46 wherein the non-motor symptoms are defined by UPDRS Version 4 part 1.
48. The method of clause 48 wherein the change in UPDRS score as defined in version 4 part 1 is at least 0.23 in comparison to a patient who did not undergo treatment with rasagiline.
49. A method of slowing clinical progression and treating symptoms of Parkinson's disease in a Parkinson's disease patient comprising periodically administering to the Parkinson's disease patient an amount of rasagiline or a pharmaceutically acceptable salt of rasagiline effective to slow clinical progression and treat the signs and symptoms of Parkinson's disease in the patient.
50. The method of clause 49, wherein the amount of rasagiline administered is 1 mg per day.
51. The method of clause 49 or 50, wherein the patient is not receiving bromocriptine, benztropine, levodopa, ropinirole, pramipexole, rotigotine, cabergoline, entacapone, tolcapone, amantadine or selegiline.
52. The method of any one of clauses 49-51, wherein the patient is not receiving any therapy for Parkinson's disease other than rasagiline or a pharmaceutically acceptable salt of rasagiline.
53. The method of any one of clauses 49-52, wherein an average Total UPDRS score of the patient increases less than 0.15 units per week after the initial symptomatic effect period of the administration of rasagiline or a pharmaceutically acceptable salt of rasagiline.
54. The method of clause 53, wherein an average Total UPDRS score of the patient increases between 0.15 and 0.05 units per week after the initial symptomatic effect period of the administration of rasagiline or a pharmaceutically acceptable salt of rasagiline.
55. The method of clause 53, wherein an average Total UPDRS score of the patient increases between 0.15 and 0.07 units per week after the initial symptomatic effect period of the administration of rasagiline or a pharmaceutically acceptable salt of rasagiline.
56. The method of clause 53, wherein an average Total UPDRS score of the patient increases between 0.11 and 0.07 units per week after the initial symptomatic effect period of the administration of rasagiline or a pharmaceutically acceptable salt of rasagiline.
57. The method of any one of clauses 49-56, wherein the increase in Total UPDRS score is less than 3.97 at 72 weeks after initiation of administration of rasagiline or a pharmaceutically acceptable salt of rasagiline.
58. The method of any one of clauses 49-56, wherein the increase in Total UPDRS score is less than 3.35 at 72 weeks after initiation of administration of rasagiline or a pharmaceutically acceptable salt of rasagiline.
59. The method of any one of clauses 49-58, wherein the pharmaceutically acceptable salt of rasagiline is rasagiline mesylate.
60. The method of any one of clauses 49-59, wherein the patient is an early stage Parkinson's disease patient.
61. The method of clause 60, wherein the early stage Parkinson's disease patient is a Stage I patient according to Hoehn and Yahr rating.
62. The method of clause 60, wherein the early stage Parkinson's disease patient is a patient whose symptoms result in a UPDRS total score of less than 25.
63. The method of clause 60, wherein the early stage Parkinson's disease patient is a patient whose symptoms result in a UPDRS motor score of less than 17.5.
64. The method of clause 60, wherein the early stage Parkinson's disease patient is a patient whose symptoms have been diagnosed to indicate Parkinson's disease within the prior 12 months.
65. The method of clause 60, wherein the early stage Parkinson's disease patient is a patient whose symptoms have been diagnosed to indicate Parkinson's disease within the prior 6 months.
66. The method of clause 60, wherein the early stage Parkinson's disease patient is a patient whose symptoms have been diagnosed to indicate Parkinson's disease within the prior 1 month.
67. The method of any one of clauses 1, 19, 25, 35 or 49, wherein the Parkinson's disease patient is a patient whose Total UPDRS score is more than 25.5.
68. The method of clause 67, wherein the amount of rasagiline administered is 1 mg per day.
69. The method of clause 68, wherein an average Total UPDRS score of the patient increases less than 0.28 units per week after the initial symptomatic effect period of the administration of rasagiline or a pharmaceutically acceptable salt of rasagiline.
70. The method of clause 68, wherein an average Total UPDRS score of the patient increases between 0.28 and 0.10 units per week after the initial symptomatic effect period of the administration of rasagiline or a pharmaceutically acceptable salt of rasagiline.
71. The method of clause 68, wherein an average Total UPDRS score of the patient increases between 0.18 and 0.10 units per week after the initial symptomatic effect period of the administration of rasagiline or a pharmaceutically acceptable salt of rasagiline.
72. The method of any one of clauses 68-71, wherein the increase in Total UPDRS score is less than 3.10 at 72 weeks after initiation of administration of rasagiline or a pharmaceutically acceptable salt of rasagiline.
73. The method of clause 67, wherein the amount of rasagiline administered is 2 mg per day.
74. The method of clause 73, wherein an average Total UPDRS score of the patient increases less than 0.28 units per week after the initial symptomatic effect period of the administration of rasagiline or a pharmaceutically acceptable salt of rasagiline.
75. The method of clause 73, wherein an average Total UPDRS score of the patient increases between 0.28 and 0.01 units per week after the initial symptomatic effect period of the administration of rasagiline or a pharmaceutically acceptable salt of rasagiline.
76. The method of clause 73, wherein an average Total UPDRS score of the patient increases between 0.09 and 0.01 units per week after the initial symptomatic effect period of the administration of rasagiline or a pharmaceutically acceptable salt of rasagiline.
77. The method of any one of clauses 73-76, wherein the increase in Total UPDRS score is less than 2.61 at 72 weeks after initiation of administration of rasagiline or a pharmaceutically acceptable salt of rasagiline.
78. Rasagiline or a pharmaceutically acceptable salt of rasagiline for use in reducing the rate of progression of Parkinson's disease symptoms in an early stage Parkinson's disease patient.
79. Rasagiline or a pharmaceutically acceptable salt of rasagiline for use in delaying the need for symptomatic anti-Parkinsonian therapy in an early stage Parkinson's disease patient.
80. Rasagiline or a pharmaceutically acceptable salt of rasagiline for use in reducing the risk of an early stage Parkinson's disease patient requiring symptomatic anti-Parkinsonian therapy.
81. Rasagiline or a pharmaceutically acceptable salt of rasagiline for use in reducing the functional decline in an early stage Parkinson's disease patient.
82. Rasagiline or a pharmaceutically acceptable salt of rasagiline for use in treating a patient exhibiting early signs of Parkinson's disease.
83. Rasagiline or a pharmaceutically acceptable salt of rasagiline for use in reducing the fatigue in an early stage Parkinson's disease patient.
84. Rasagiline or a pharmaceutically acceptable salt of rasagiline for use in reducing the severity of non-motor symptoms in an early stage Parkinson's disease patient.
85. Rasagiline or a pharmaceutically acceptable salt of rasagiline for use in slowing clinical progression and treating symptoms of Parkinson's disease in a Parkinson's disease patient.
86. A pharmaceutical composition comprising a pharmaceutically effective amount of rasagiline or a pharmaceutically acceptable salt of rasagiline for use in reducing the rate of progression of Parkinson's disease symptoms in an early stage Parkinson's disease patient.
87. A pharmaceutical composition comprising a pharmaceutically effective amount of rasagiline or a pharmaceutically acceptable salt of rasagiline for use in delaying the need for symptomatic anti-Parkinsonian therapy in an early stage Parkinson's disease patient.
88. A pharmaceutical composition comprising a pharmaceutically effective amount of rasagiline or a pharmaceutically acceptable salt of rasagiline for use in reducing the risk of an early stage Parkinson's disease patient requiring symptomatic anti-Parkinsonian therapy.
89. A pharmaceutical composition comprising a pharmaceutically effective amount of rasagiline or a pharmaceutically acceptable salt of rasagiline for use in reducing the functional decline in an early stage Parkinson's disease patient.
90. A pharmaceutical composition comprising a pharmaceutically effective amount of rasagiline or a pharmaceutically acceptable salt of rasagiline for use in treating a patient exhibiting early signs of Parkinson's disease.
91. A pharmaceutical composition comprising a pharmaceutically effective amount of rasagiline or a pharmaceutically acceptable salt of rasagiline for use in reducing the fatigue in an early stage Parkinson's disease patient.
92. A pharmaceutical composition comprising a pharmaceutically effective amount of rasagiline or a pharmaceutically acceptable salt of rasagiline for use in reducing the severity of non-motor symptoms in an early stage Parkinson's disease patient.
93. A pharmaceutical composition comprising a pharmaceutically effective amount of rasagiline or a pharmaceutically acceptable salt of rasagiline for use in slowing clinical progression and treating symptoms of Parkinson's disease in a Parkinson's disease patient.

## Claims

1. Rasagiline, or a pharmaceutically acceptable salt of rasagiline for use in reducing the rate of progression of Parkinson's disease in an early stage Parkinson's disease patient, comprising periodic administration to an early stage Parkinson's disease patient an amount of rasagiline, or a pharmaceutically acceptable salt of rasagiline effective to reduce the rate of progression of Parkinson's disease of the early stage Parkinson's disease patient.

2. Rasagiline, or a pharmaceutically acceptable salt of rasagiline of claim 1, wherein the amount of rasagiline administered is 1 mg per day.

3. Rasagiline, or a pharmaceutically acceptable salt of rasagiline any one of claims 1-2, wherein the pharmaceutically acceptable salt of rasagiline is rasagiline mesylate.

4. Rasagiline, or a pharmaceutically acceptable salt of rasagiline for use in reducing the rate of progression of Parkinson's disease in a Parkinson's disease patient, comprising periodic administration to the Parkinson's disease patient for more than 52 weeks an amount of rasagiline, or a pharmaceutically acceptable salt of rasagiline effective to reduce the rate of progression of Parkinson's disease.

5. Rasagiline, or a pharmaceutically acceptable salt of rasagiline for use in delaying the need for symptomatic anti-Parkinsonian therapy in an early stage Parkinson's disease patient, comprising periodic administration to an
early stage Parkinson's disease patient an amount of rasagiline or a pharmaceutically acceptable salt of rasagiline effective to delay the need for symptomatic anti-Parkinsonian therapy.

6. Rasagiline, or a pharmaceutically acceptable salt of rasagiline of claim 5, wherein the amount of rasagiline administered is 1 mg per day.

7. Rasagiline, or a pharmaceutically acceptable salt of rasagiline any one of claims 5-6, wherein the pharmaceutically acceptable salt of rasagiline is rasagiline mesylate.

8. Rasagiline, or a pharmaceutically acceptable salt of rasagiline for use in reducing the risk of a Parkinson's disease patient requiring symptomatic anti-Parkinsonian therapy, comprising periodic administration to the patient for 36 weeks an amount of rasagiline or a pharmaceutically acceptable salt of rasagiline effective to reduce the risk of requiring symptomatic anti-Parkinsonian therapy.

9. Rasagiline, or a pharmaceutically acceptable salt of rasagiline for use in reducing the functional decline of an early stage Parkinson's disease patient, comprising identifying a patient to be an early stage Parkinson's disease patient, and periodically administering to the patient so identified an amount of rasagiline or a pharmaceutically acceptable salt of rasagiline effective to reduce the functional decline.

10. Rasagiline, or a pharmaceutically acceptable salt of rasagiline for use in reducing the functional decline in a Parkinson's disease patient, comprising periodic administration to the patient for more than 52 weeks an amount of rasagiline or a pharmaceutically acceptable salt of rasagiline effective to reduce the functional decline.

11. Rasagiline, or a pharmaceutically acceptable salt of rasagiline for use in treating a patient exhibiting early signs of Parkinson's disease, comprising identifying a patient exhibiting early signs of Parkinson's disease, and periodically administering to the patient so identified an amount of rasagiline or a pharmaceutically acceptable salt of rasagiline effective to treat the patient.

12. Rasagiline, or a pharmaceutically acceptable salt of rasagiline for use in reducing fatigue in an early stage Parkinson's disease patient, comprising periodic administration to an early stage Parkinson's disease patient an amount of rasagiline or a pharmaceutically acceptable salt of rasagiline effective to reduce fatigue.

13. Rasagiline, or a pharmaceutically acceptable salt of rasagiline for use in reducing severity of non-motor symptoms in an early stage Parkinson's disease patient, comprising periodic administration to an early stage Parkinson's disease patient an amount of rasagiline or a pharmaceutically acceptable salt of rasagiline effective to reduce the severity of non-motor symptoms.

14. Rasagiline, or a pharmaceutically acceptable salt of rasagiline for use in slowing clinical progression and treating symptoms of Parkinson's disease in a Parkinson's disease patient comprising periodic administration to the Parkinson's disease patient an amount of rasagiline or a pharmaceutically acceptable salt of rasagiline effective to slow clinical progression and treat the signs and symptoms of Parkinson's disease in the patient.

15. A pharmaceutical composition comprising a pharmaceutically effective amount of rasagiline or a pharmaceutically acceptable salt of rasagiline for use in reducing the rate of progression of Parkinson's disease symptoms in an early stage Parkinson's disease patient,
in delaying the need for symptomatic anti-Parkinsonian therapy in an early stage Parkinson's disease patient;
in reducing the risk of an early stage Parkinson's disease patient requiring symptomatic anti-Parkinsonian therapy;
in reducing the functional decline in an early stage Parkinson's disease patient;
in treating a patient exhibiting early signs of Parkinson's disease;
in reducing the fatigue in an early stage Parkinson's disease patient;
in reducing the severity of non-motor symptoms in an early stage Parkinson's disease patient; or
in slowing clinical progression and treating symptoms of Parkinson's disease in a Parkinson's disease patient.
